# EUROPÄISCHE PATENTSCHRIFT

(11) **EP 1 149 280 B1**
(45) Veröffentlichungstag und Bekanntmachung des Hinweises auf die Patenterteilung: **30.10.2002**
(21) Anmeldenummer: 99948861.2
(22) Anmeldetag: 24.09.1999
(51) Int. Cl.: G01N 21/64, G01N 33/58

(54) **VERFAHREN UND VORRICHTUNG FÜR AUTOMATISIERTE CHROMOPHOR-UNTERSTÜTZTE LASER/LICHT-INAKTIVIERUNG (CALI)**
METHOD AND DEVICE FOR AUTOMATED CHROMOPHORE-ASSISTED LASER/LIGHT INACTIVATION (CALI)
PROCEDE ET DISPOSITIF D'INACTIVATION AUTOMATISEE, AU MOYEN D'UNE LUMIERE/D'UN LASER, ASSISTEE PAR DES CHROMOPHORES

(30) Priorität: 24.11.1998 DE 19854195
(43) Veröffentlichungstag der Anmeldung: 31.10.2001
(73) Patentinhaber: Xerion Pharmaceuticals AG, 82152 Martinsried (DE)
(72) Erfinder: RUDERT, Fritz, H., D-81241 München (DE); RUDOLPH, Peter, D-80686 München (DE); BECK, Stefan, D-80339 München (DE)
(74) Vertreter: Bösl, Raphael, Dr. rer. nat., Dipl.-Chem.
(86) Internationale Anmeldenummer: EP9907126
(87) Internationale Veröffentlichungsnummer: WO00031517

(56) Entgegenhaltungen:
- EP-A- 0 841 557
- WO-A-98/38490
- DE-A- 3 818 654
- US-A- 5 780 857
- D.G. JAY: "Selective destruction of protein function by chromophore-assisted laser inactivation" PROCEEDINGS OF THE NATIONAL ACADEMY OF SCIENCES OF USA., Bd. 85, August 1988 (1988-08), Seiten 5454-5458, XP000857275 NATIONAL ACADEMY OF SCIENCE. WASHINGTON., US ISSN: 0027-8424 in der Anmeldung erwähnt
- T. SURREY ET AL.: "Chromophore-assisted light inactivation and self-organization of microtubules and motors" PROCEEDINGS OF THE NATIONAL ACADEMY OF SCIENCES OF USA., Bd. 95, April 1998 (1998-04), Seiten 4293-4298, XP000857273 NATIONAL ACADEMY OF SCIENCE. WASHINGTON., US ISSN: 0027-8424 in der Anmeldung erwähnt

## Beschreibung

Die Erfindung bezieht sich auf eine Vorrichtung für die automatisierte Chromophor-unterstützte Laser/Licht-Inaktivierung (CALI) sowie auf ein Verfahren zur Identifikation der Funktion biologischer Moleküle mittels Chromophor-unterstützter Licht-Inaktivierung.

Im Stand der Technik ist die Chromophor-unterstützte Licht-Inaktivierung auf der Basis eines Lasers als Lichtquelle bekannt geworden. Die Chromophor-unterstützte Laser-Inaktivierung wurde erstmalig in Jay, D.G., Selective Destruction of Protein Function By Chromophore-Assisted Laser Inactivation, Proceedings of National Academy of Science, USA 85 (15):5454-8, beschrieben. Diese bekannte Technik kann selektiv zur zeitlich und räumlich auflösenden Inaktivierung von Proteinen eingesetzt werden. Mit Hilfe dieser Technik wird dabei ein Farbstoff, welcher normalerweise das Chromophor Malachitgrün (MG) ist, chemisch an einen Antikörper gekoppelt, welcher Spezifität für ein Zielmolekül besitzt. Nach erfolgter Bindung des MG-Antikörpers wird der Komplex mit gepulstem, hochenergetischem Laserlicht beschossen. Dies führt lokal über das MG zur Bildung von reaktiven, kurzlebigen (10⁻¹¹s) Hydroxylradikalen, welche das Zielmolekül modifizieren und dadurch zu dessen Inaktivierung führen können. Dies ist beschrieben in Liao, J.C., et al., Chormophore-Assisted Laser Inactivation of Proteins Is Mediated by the Photogeneration of Free Radicals, in Proceedings of the National Academy of Science, USA 91 (7):2659-63. In jüngster Zeit sind für die Chromophor-unterstützte Laser-Inaktivierung außer MG auch der Fluoreszenzfarbstoff Fluorescein (FITC) und ein Laser mit kontinuierlicher Welle, d.h. ein kontinuierlicher Laser, erfolgreich eingesetzt worden. Dies ist beschrieben in Surrey, T. et al., Chormophore-Assisted Light Inactivation and Self-Organiszation of Microtubules And Motors, Proceedings of the National Academy of Science, USA 95:4293-8. Dabei zeigte sich, daß FITC im Vergleich zu MG potenter ist und schneller zur Zielmolekül-Inaktivierung führt. Dabei zeigte sich auch, daß bei Verwendung von FITC auch deutlich weniger leistungsstarke kontinuierliche Laser zu einem vergleichbaren Effekt erforderlich waren wie wesentlich leistungsstärkere gepulste oder kontinuierliche Laser bei Verwendung von MG. Daraus ist ersichtlich, daß CALI noch breite Entwicklungsspielräume bietet und damit verbesserungsfähig und verbesserungswürdig ist. Unter dem Begriff Chromophore werden im Sinne dieser Anmeldung auch Fluorophore verstanden.

Zum Beispiel sind eine ganze Reihe weiterer Farbstoffe CALI-tauglich oder können sich sogar den bisher benutzten Farbstoffen als überlegen zeigen.

Des weiteren wurde der gesamte CALI-Vorgang bisher nahezu ausschließlich manuell ausgeführt, wodurch der Durchsatz gering war, eine Standardisierungsmöglichkeit außerordentlich begrenzt war, menschliche Fehlerquellen eingeschlossen waren sowie Gefahren für den Experimentator bestanden.

Des weiteren kam CALI überwiegend in seiner Mikrovariante für die Inaktivierung von Zielmolekülen auf Einzelzellniveau mittels eines mikroskopisch fokussierten Laserstrahls zum Einsatz. Um jedoch die vollen Möglichkeiten der Makrovariante von CALI beispielsweise für die Protein-Inaktivierung in massiven Screening-Ansätzen ausschöpfen zu können, ist es wünschenswert, den gesamten Vorgang zu automatisieren, zu standardisieren und kostengünstig zu gestalten.

Im einzelnen ist in dem zitierten Artikel von Jay et al. sowie bei Lindan, K. G. et al., Spacial Specificity of Chromophore-Assisted Laser Inactivation of Protein Function, in Biophys. J. 61 (4): 956-62, die Verwendung eines Farbstofflasers in Form eines Quanta Ray DCR3 (z.B. von Spectra Physics Inc., Mountain View, CA) Nd:YAG (Neodynium-Yttrium-Aluminium Garnet), ein gepulster Infrarotlaser mit 10 Hz, 8,5 nm Pulslänge und 1064 Nanometer Wellenlänge, dargestellt, um mit dessen zweiter harmonischer Wellenlänge (532 nm Wellenlänge) einen nachgeschalteten Farbstofflaser (Quanta Ray PDL2, von Spectra Physics) zu betreiben, welcher den zirkulierenden, fluoreszierenden Laserfarbstoff DCM (Exciton Chemical CO., Dayton, OH) enthielt. Das generierte, gepulste Laserlicht von 620 nm liegt in der Nähe des Absorptionsmaximums von Malachitgrün. Dieser Strahl wurde mittels eines Rechtwinkelprismas durch Totalreflexion in die Vertikale umgelenkt und durch eine Konvexlinse auf den gewünschten Durchmesser von ca. 2 mm fokussiert. Die Ausgabeenergie des Laserstrahls mit 620 nm wurde in CALI-Experimenten bei 10 bis 15 mJ /Puls mit einer Pulsfrequenz von 10 Hz festgelegt. Dies entsprach einer durchschnittlichen Spitzenleistungsdichte pro Puls von 37 bis 57 MW/cm² und einer durchschnittlichen Laserausgangsleistung von ca. 80 bis 100 mW. Es wurde ein molares Verhältnis von ca. 20:1 für MG-gekoppelte Antikörper (100 µg/ml) zu ß-Galaktosidase-Zielmolekül (5,5 µg/ml) eingesetzt. Das Kopplungsverhältnis für den Antikörper betrug ca. 6,5 MG Moleküle pro Antikörper. Die halbmaximale Inaktivierungszeit von β-Galaktosidase betrug unter diesen Bedingungen ca. 150 s.

Des weiteren ist in dem oben beschriebenen Artikel von Surrey et al. beschrieben, daß ein Argon-gepumpter einstellbarer (justierbarer) Farbstofflaser mit kontinuierlicher Welle (coherent 599 standing wave dye laser with rhodamine 6G, Coherent Radiation, Palo Alto, CA) für CALI Malachitgrün benutzt wurde. Die erzeugten Wellenlängen betrugen 620 nm und 488 nm, wobei die Wellenlänge von 488 nm im Bereich des zweiten Absorptionsmaximum von MG liegt, bei einer Leistung von 225 mW. Eine konstante Leistungsdichte von 7,2 W/cm² wurde zur Inaktivierung von β-Galaktosidase in vitro benutzt. Es wurde ein molares Verhältnis von ca. 20:1 für MG-gekoppelten Antikörper (200 µg/ml) zu β-Galaktosidase-Zielmolekül (10 µg/ml) eingesetzt. Das Kopplungsverhältnis für den Antikörper betrug ca. 2,8 MG-Moleküle pro Antikörper. Die halbmaximale Inaktivierungszeit von β-Galaktosidase betrug unter diesen Bedingungen 8,5 s bei einer Wellenlänge von 620 nm und 13 s bei einer Wellenlänge von 488 nm. Ein schwächerer ArgonIonenlaser mit einer kontinuierlichen Welle einer Wellenlänge von 488 nm und einer konstanten Leistung von 2 mW wurde für CALI mit FITC verwendet. Dies entsprach einer konstanten Leistungsdichte von 0,065 W/cm². Es wurde ein molares Verhältnis von ca. 20 : 1 für MG-gekoppelten Antikörper (10 µg/ml) zu β-Galaktosidase-Zielmolekül (5,5 µg/ml) eingesetzt. Das Kopplungsverhältnis für den Antikörper betrug ca. 2,3 FITC-Moleküle pro Antikörper. Die halbmaximale Inaktivierungszeit von β-Galaktosidase betrug unter diesen Bedingungen 18 s.

Des weiteren ist in Jean, B. et al., Selective Laser-Induced Inactivation of Proteins (SLIP) by Labelling with Chromophores, 1992, Med. & Biol. Eng. & Comput., 30, CE17-CE20, beschrieben, daß ein Argon-gepumpter einstellbarer (justierbarer) Farbstofflaser mit einer kontinuierlichen Welle (Meditech Dye Laser With Rhodamine 6G) für SLIP (Selective Laser-Induced Inactivation of Proteins) mit FITC bei einer Wellenlänge von 488 nm benutzt wurde. Die Proben wurden mit einer Leistung von 3 W bestrahlt. Im Falle von SLIP wurde der Farbstoff direkt an das zu inaktivierende Molekül gekoppelt (Antikörper und Enzym Meerrettich-Peroxidase). Die Kopplungseffizienzen lagen zwischen 1:1 und 2:1. Es wurden 8 bis 10 Pulse von 1 s Dauer gewählt. Dies führte zu einer halbmaximalen Inaktivierung von Meerretlich-Peroxidase bei einer Energiedichte von ca. 7500 J/cm². Dieser Wert erscheint ungewöhnlich hoch, da zum Vergleich etwa während 150 s halbmaximaler Inaktivierungszeit mit einem gepulsten Hochleistungs-Nd:YAG-Laser lediglich ca. 480 J/cm² auf die Probe einwirken.

EP-A-0 841 557 beschreibt eine Vorrichtung für die automatisierte Laseranregung von z.B. mit Fluoreszensmarkern markierten Biomolekülen, die jedoch keinen Leistungsmesser zur Bestimmung der von der Laservorrichtung abgegebenen Leistung aufweist.

US-A-5 780 857 offenbart eine Vorrichtung zur Laserbestrahlung von planaren biochemischen Proben mit hoher Sensitivität. Es ist jedoch kein Leistungsmesser für die Einstellung und Überwachung der Leistung entsprechend der jeweiligen Probenart vorgesehen.

Im Hinblick auf die bekannten Gerätesysteme liegt der vorliegenden Erfindung die Aufgabe zugrunde, ein flexibles, kostengünstiges, höchst zuverlässiges automatisierbares CALI-System/-Verfahren zu schaffen, welches eine hohe Durchsatzleistung hinsichtlich der zu inaktivierenden Proben ermöglicht und insbesondere flexibel im Hinblick auf die eingesetzte Lichtquelle ist. Diese Aufgabe wird durch eine Vorrichtung mit den Merkmalen gemäß Anspruch 1 bzw. 6 und durch ein Verfahren mit den Merkmalen gemäß Anspruch 19 gelöst.

Zweckmäßige Weiterbildungen sind in den jeweiligen abhängigen Ansprüchen definiert.

Die Vorrichtung für die automatisierte Chromophor-unterstützte Laser-Inaktivierung gemäß der Erfindung weist eine Laservorrichtung zur Bestrahlung von Proben mittels eines Laserstrahls zur Identifizierung der Funktion von biologischen Molekülen, eine Steuereinrichtung zur Steuerung von beispielsweise mechanischen, optischen und thermischen Parametern der Vorrichtung und zumindest einen Probenhalter auf. Mindestens ein Antrieb ist vorgesehen, um eine Relativbewegung zwischen dem aus der Laservorrichtung austretenden Laserstrahl und dem Probenhalter in zumindest zwei, vorzugsweise senkrecht zueinander stehenden, Richtungen zu erzeugen. Mittels dieser Relativbewegung werden die Proben mit dem Laserstrahl bestrahlt bzw. sind über diesen Laserstrahl abtastbar. Erfindungsgemäß ist der Laserstrahl mittels einer Strahlmodifikations-vorrichtung in seiner Form und/oder Intensität an die Probengröße und/oder die Probenart und/oder die Probenanzahl anpaßbar. Um diese Anpassung zu realisieren, ist es möglich, als Strahlmodifikationseinrichtung eine Strahlaufweitvorrichtung (beam expander) und/oder einen Strahlteiler (beam splitter) einzusetzen. Mit dem Strahlteiler wird der Lichtstrahl so aufgeteilt, daß im Interesse eines hohen Durchsatzes zu behandelnder Proben mehrere Proben gleichzeitig bestrahlbar sind. Die Steuereinrichtung ist gemäß der Erfindung so ausgebildet, daß der Antrieb für die Relativbewegung zwischen dem aus der Laservorrichtung austretenden Laserstrahl und dem Probenhalter und der Antrieb für die Strahlaufweitvorrichtung bzw. den Strahlteiler mittels dieser Steuereinrichtung zentral mittels einer Systemsoftware steuerbar sind.

Mit der erfindungsgemäßen Vorrichtung wird der automatisierte CALI-Vorgang hinsichtlich der in einer Zeiteinheit zu behandelnden Proben erheblich beschleunigt und optimiert, werden seine Kosten beträchtlich reduziert und wird gleichzeitig seine Genauigkeit der Einstellung im Hinblick auf das Ausschließen menschlicher Fehlerquellen und damit die Sicherheit und Reproduzierbarkeit der Ergebnisse erhöht. Durch das Verwenden einer einzigen Software zur Steuerung zumindest der wesentlichen Teile der CALI-Vorrichtung wird eine erhebliche Vereinfachung in der Funktion und im Betreiben der Vorrichtung erreicht.

Durch Verwenden einer Strahlaufweitvorrichtung ist es möglich, die Intensität und den Querschnitt des Lichtstrahls nicht nur an die jeweilige Probe anzupassen, sondern auch an unterschiedliche Abmessungen der Kavitäten für die einzelnen Proben in den Probenhaltern. Damit wird eine hohe Flexibilität des Einsatzes für unterschiedlichste Probengrößen und unterschiedlichste Untersuchungszwecke erreicht.

Bei Verwendung eines Stahlteilers bzw. bei Modifikation der Strahlaufweitvorrichtung derart, daß der Lichtstrahl in mehrere einzelne Lichtstrahlen aufgeteilt wird, wie es bei Verwendung eines ebenfalls an sich bekannten Strahlteilers der Fall ist, ist es möglich, mehrere Proben parallel der Protein-Inaktivierung auszusetzen, wodurch der Durchsatz der erfindungsgemäßen CALI-Vorrichtung weiter erhöht werden kann.

Vorzugsweise weitet die Strahlaufweitvorrichtung den Laserstrahl im Querschnitt eindimensional, insbesondere als gerade Linie, oder zweidimensional als Fläche unterschiedlicher Konfiguration auf, wobei die Querschnittsfläche des Laserstrahls vorzugsweise rechteckig, kreisförmig oder ellipsenförmig ist. Diese Flexibilität im Querschnitt dient dem Einsatz für unterschiedliche, im Probenhalter vorgesehene, die Probe aufnehmende Kavitäten.

Gemäß einer bevorzugten Ausgestaltung ist die Strahlaufweitvorrichtung so ausgebildet, daß zumindest zwei Proben gleichzeitig bestrahlbar sind, wobei dies entweder durch einen entsprechend aufgeweiteten Laserstrahl oder durch Aufteilung des Laserstrahls nach Verlassen der Strahlaufweitvorrichtung in zwei separate Strahlen möglich ist. Dabei ist jedoch zu beachten, daß die Anzahl der die Strahlaufweitvorrichtung bzw. den Strahlteiler verlassenden Strahlen von der Leistung des Lasers abhängig ist, wobei höhere erforderliche Leistungen des eingesetzten Lasers mit höheren Kosten der Vorrichtung verbunden sind.

Gemäß einer Weiterbildung der Erfindung ist die Steuereinrichtung so ausgebildet, daß sie den die Laservorrichtung verlassenden Strahl in seinem Querschnitt, seiner Intensität, in der Bestrahlungsdauer und der Abmessung in Abhängigkeit von der Bewegung des Probenhalters und der Anzahl und/oder der Art der Proben variiert. Die Abstimmung zwischen Parametern des Laserstrahls und Relativbewegung zwischen Laserstrahl und Probenhalter dient ebenfalls der Erhöhung des Durchsatzes der CALI-Vorrichtung sowie der Erhöhung der Genauigkeit und Reproduzierbarkeit der Ergebnisse aufgrund eines exakten Timings.

Gemäß einer bevorzugten Ausführungsform ist die Lichtquellenvorrichtung eine Laservorrichtung, und zwar vorzugsweise ein gepulster Laser, insbesondere ein Nd:YAG-Infrarot-Laser. Zum Betreiben eines optischen parametrischen Oszillators (Infinity-XPO, Coherent Radiation) zur Wellenlängenänderung wird die dritte harmonische Wellenlänge des gepulsten Infrarot-Lasers verwendet.

Gemäß einer weiteren Ausführungsform ist die Laservorrichtung ein kontinuierlicher Laser, und zwar insbesondere ein Argon-gepumpter justierbarer Farbstofflaser. Es ist jedoch auch möglich, einen Argon-Ionen-Gas-Laser oder einen Helium-Neon-Laser zu verwenden.

Gemäß einem weiteren Ausführungsbeispiel der Erfindung erfolgt die Bestrahlung von Proben zur Identifikation der Funktion von biologischen Molekülen mittels aus einer Lichtquellenvorrichtung ausgesendeten Lichtes auf der Basis einer automatisierten Chromophor-unterstützten Licht-Inaktivierung (CALI = Chromophore-Assisted Light Inactivation). Die Vorrichtung weist eine Steuereinrichtung zur zentralen Steuerung aller zu steuernden Elemente/Baugruppen der Vorrichtung und zumindest einen Probehalter auf. Erfindungsgemäß ist die Lichtquellenvorrichtung ein Beleuchtungskörper, bei welchem zumindest ein Teil der Wellenlänge seines ausgesendeten Lichts im sichtbaren Bereich liegt. Als Beleuchtungskörper wird vorzugsweise eine Glühlampe, Quarzlampe, Leuchtstoffröhre oder Leuchtdiode oder eine Kombination aus diesen eingesetzt, wobei der Beleuchtungskörper insbesondere eine Weißlichtquelle ist. Das bedeutet, daß beispielsweise eine handelsübliche Glühlampe unter Verwendung geeigneter Chromophore in der Lage ist, die ansonsten gemäß dem ersten Ausführungsbeispiel erforderliche Inaktivierung biologischer Moleküle zu erzielen, ohne daß eine Laservorrichtung Anwendung finden muß.

Vorzugsweise ist das durch die Lichtquellenvorrichtung ausgesendete Licht mittels einer Strahlmodifikationseinrichtung in seiner davon ausgesandten Form und/oder Intensität an verschiedene Parameter der Proben anpaßbar. Insbesondere sind dies die Probengröße, die Probenart bzw. die Probenanzahl, wobei das ausgesendete Licht an einen, mehrere oder alle aufgeführten Probenparameter anpaßbar ist. Damit wird die Flexibilität der erfindungsgemäßen Vorrichtung enorm erhöht, weil unterschiedlichste Proben mit hoher Durchsatzleistung, großer Zuverlässigkeit und sehr großer Bandbreite untersuchbar sind.

Des weiteren ist vorzugsweise gemäß einer Weiterbildung der Erfindung eine Steuereinrichtung vorgesehen, mittels welcher ein Antrieb zur Bewegung des Probenhalters und die Strahlmodifikationseinrichtung zentral über eine Systemsoftware steuerbar sind. Die Steuereinrichtung ist dafür vorgesehen, mittels der zentralen Systemsoftware sämtliche steuerbaren Komponenten der erfindungsgemäßen Vorrichtung zu steuern. Vorzugsweise weist die Strahlmodifikationseinrichtung Masken, insbesondere mit variablen Öffnungen/Blenden auf. Die Masken mit den variablen Öffnungen bzw. Blenden dienen dazu, das ausgesendete Licht gezielt auf mehrere auf dem Probenhalter angeordnete Proben gleichzeitig zur Bestrahlung zu richten, wobei vorzugsweise auch alle auf einem Standardprobenhalter in Form einer Mikrotiterplatte angeordneten vorzugsweise 96 Proben gleichzeitig bestrahlbar sind, wodurch extrem hohe Durchsätze durch die erfindungsgemäße Vorrichtung möglich sind.

Vorzugsweise ist das Licht zumindest ein Lichtstrahl. Gemäß einer Weiterbildung der Erfindung ist es jedoch auch möglich, daß gemäß einer besonders bevorzugten Ausführungsform der Erfindung das Licht diffuses Licht ist. Bei Verwendung von diffusem Licht sind optische Einrichtungen zwischen der Lichtquellenvorrichtung und den Proben in der Regel nicht erforderlich, sondern das diffus ausgesendete Licht wird zur gleichzeitigen Bestrahlung einer möglichst großen Anzahl von auf dem Probenhalter angeordneter Proben benutzt. Bei Verwendung einer solchen diffusen Weißlichtquelle ist eine Einzelstrahlteilung nicht erforderlich, wobei im Falle einer erforderlichen Beschränkung der Bestrahlung auf bestimmte Proben, um nicht sämtliche Proben gleichzeitig bestrahlen zu müssen, die Verwendung geeigneter Lochmasken vorgesehen ist. Das bedeutet, daß mit der Verwendung einer diffusen Weißlichtquelle eine besonders hohe Parallelisierung der Probenbestrahlung unter Verwendung einer Lochmaskenblende erreichbar ist. Diese Parallelisierung der Probenbestrahlung ist besonders hoch im Vergleich zum fokussierten Laserstrahl gemäß dem ersten Ausführungsbeispiel, bei welchem ein sukzessives Abtasten der einzelnen, in Kavitäten auf einer Mikrotiterplatte auf dem Probenhalter angeordneten Proben erfolgt.

Bei Verwendung einer an sich bekannten Weißlichtglühlampe kann die erfindungsgemäße Vorrichtung besonders einfach im Aufbau und vor allem auch kostengünstig bereitgestellt werden. Bei Verwendung einer Weißlichtglühlampe ist entweder die diffuse, gleichzeitige Bestrahlung vieler Proben über eine Lochmakse möglich, oder es ist mittels relativ einfacher konstruktiver optischer Baugruppen möglich, das von der Lichtquellenvorrichtung ausgesendete Licht nach einer entsprechenden Bündelung wieder so zu teilen, daß eine möglichst große Anzahl von Proben gleichzeitig bestrahlbar wird. Eine Bestrahlung auch ohne optische Baugruppen ist ebenfalls möglich. Ein weiterer Vorteil der Verwendung von Weißlichtglühlampen zur Bestrahlung besteht unter anderem darin, daß Gefahrenquellen fiir Bedienpersonen im Vergleich zur Verwendung einer Laservorrichtung als Lichtquellenvorrichtung deutlich reduziert werden.

Für die jeweiligen Proben ist unter Beachtung der Tatsache, ob eine Strahlaufweitvorrichtung und/oder ein Strahlteiler im Zusammenhang mit einer Laservorrichtung oder mit einer Weißlichtglühlampe vorgesehen ist, d. h., um die für die jeweiligen Proben erforderlichen Leistungen bzw. Lichtausbeuten der jeweils verwendeten Lichtstrahlen einstellen zu können, ein Leistungsmesser zur Bestimmung der von der Lichtquellenvorrichtung abgegebenen Leistung vorgesehen. Mit einem derartigen Leistungsmesser ist somit auch eine höhere Reproduzierbarkeit der Untersuchungsbedingungen gegeben.

Um eine hohe Flexibilität bezüglich der Relativbewegung zwischen Lichtstrahl und Probenhalter sowie um ein rasches Bestrahlen der am Probenhalter angebrachten Mikrotiterplatten mit den entsprechenden Proben zu gewährleisten, weist der Probenhalter einen Positioniertisch auf, welcher zumindest eine Mikrotiterplatte trägt, vorzugsweise jedoch zwei Mikrotiterplatten aufweist, und welcher in x,y-Richtung positionierbar ist. Die Positionierung in x,y-Richtung wird mittels rechtwinklig zueinander angeordneter Verschiebemechanismen tealisiert, welche mittels Schrittmotoren angetrieben sind. Die Schrittmotoren basieren auf Vollschritt- oder Halbschritt-Technologie und erlauben ein sehr genaues, im Zusammenhang mit den entsprechenden Verschiebemechanismen auch weitgehend spielfreies Einstellen exakt gewünschter Positionen des Positioniertisches mit den Mikrotilerplatten. Ein derartiger x,y-Positioniertisch ist insbcsondere bei Verwendung einer Laservorrichtung als Lichtquellenvorrichtung erforderlich.

Vorzugsweise sind die Verschiebemechanismen schienengeführt, wobei zur Übertragung der Bewegung vom jeweiligen Schrittmotor Kugelspindeln vorgesehen sind, welche eine hohe Positioniergenauigkeit im Hinblick auf die durch den elektronisch angesteuerten Schrittmotor verursachte Bewegung realisieren.

Die erfindungsgemäße Vorrichtung stellt den zentralen Teil eines CALI-Systems zur Identifizierung der Funktion von biologischen Molekülen dar und umfaßt gemäß einer weiteren Ausführungsform der Erfindung zur weiteren Erhöhung der Flexibilität des Gesamtsystems noch zusätzlich eine Liganden-Bindungspartner(LBP)-Screening-Maschine zur Erzeugung spezifischer, gegen spezielle Zielmoleküle/Liganden gerichtete LBPs, ggf. eine Chromophor-Synthesevorrichtung zur Produktion von Chromophoren, eine LBP-Chromophor-Kopplungsvorrichtung zur Verknüpfung der ausgewählten LBPs und der synthetischen Chromophore, eine Beladungsvorrichtung zum Überführen von LBP-Tags in in der Mikrotiterplatte angeordnete Kavitäten, eine Einrichtung zum Bewegen der Mikrotiterplatte in die CALI-Vorrichtung und eine Vorrichtung zum Ablesen der Aktivität der zu inaktivierenden Zielmoleküle.

Eine diese genannten weiteren Baugruppen umfassende Vorrichtung stellt damit eine Vorrichtung mit hoher Flexibilität dar, welche eine weitestgehende automatisierte Beschickung, Bestrahlung, Untersuchung und Austausch bereits untersuchter gegen neue Proben ermöglicht und des weiteren auch ein automatisiertes Ablesen und Abspeichern der durch die Untersuchung gewonnenen Ergebnisse. Insbesondere durch die Verwendung eines zentralen Steuersystems für sämtliche Komponenten des Gesamtsystems wird ein leichtes und übersichtliches Handling sowie ein optimales Betreiben der gesamten Vorrichtung ermöglicht.

Um neugewonnene Ergebnisse mit bisherigen Ergebnissen vergleichen zu können, um Trends der Untersuchung von bestimmten Proben rechtzeitig erkennen zu können und um auch später Zugriff auf einmal gewonnene Untersuchungsergebnisse zu haben, werden die Ableseergebnisse in einer Datenbank abgespeichert.

Neben der Steuerung der Screening-Maschine, der LBP-Chromophor-Kopplungsvorrichtung, der Beladungsvorrichtung und der Einrichtung zum Bewegen der Mikrotiterplatte wird auch ein weiterer Antrieb zur Bewegung einer zwischen der Strahlmodifikationsvorrichtung und dem Probenhalter angeordneten optischen Linse mittels der Steuereinrichtung über die Systemsoftware zentral gesteuert. Dadurch wird die Flexibilität des Gesamtsystems weiter erhöht, wobei gleichzeitig seine Zuverlässigkeit steigt.

Gemäß einer bevorzugten Ausführungsform der Erfindung ist ein Gehäuse zumindest um eine Mikrotiterplatte des Probenhalters derart angeordnet, daß es diese umschließt, vorzugsweise jedoch um den gesamten Probenhalter samt Bewegungsmechanik angeordnet. Das Gehäuse ist dabei mit äußeren Versorgungssystemen derart verbunden, daß bestimmte, vorher eingestellte gewünschte Zustandsbedingungen im Gehäuse steuerbar sind. Diese Zustandsbedingungen sollen insbesondere Zustandsbedingungen der die jeweilige Probe bzw. Mikrotiterplatte umgebenden Atmosphäre umfassen. Dazu zählen insbesondere der Kohlendioxidgehalt, die Feuchtigkeit, die Temperatur, der Druck usw. der Atmosphäre. Unter Versorgungssystemen sei im folgenden die Versorgung über spezielle zentrale oder dezentrale Leitungssysteme mit entsprechenden Ventilen verstanden, wobei die Ventile bzw. auch in den Versorgungssystemen vorgesehene Pumpen so steuerbar sind, daß die Zustandsbedingungen auf einen bestimmten Wert oder entsprechend einer gewünschten Änderungskurve einstellbar bzw. auf bestimmte Zustandswerte einregelbar sind. Vorzugsweise sind die entsprechenden Pumpen bzw. Ventile in den Versorgungssystemen ebenfalls über die Steuereinrichtung über die Systemsoftware zentral steuerbar. Zur Flexibilitätserhöhung der Gesamtanlage werden die Mikrotiterplatten mit Kavitäten unterschiedlicher Abmessungen verwendet, so daß Proben unterschiedlichster Konsistenz und unterschiedlichster Zusammensetzung je nach Bedarf angeordnet werden können.

Gemäß einem weiteren Aspekt der Erfindung wird für das erfindungsgemäße Verfahren zur Identifikation der Funktion eines Liganden zur für eine Inaktivierung erforderlichen Bestrahlung ein Beleuchtungskörper, insbesondere in Form einer Weißlichtquelle, eingesetzt. Bei dem erfindungsgemäßen Verfahren wird eine Probe mit einem Komplex aus einem Liganden und einem Ligandenbindungspartner (LBP)-lichtaktiverbaren-Marker (Tag) mit einem Lichtstrahl aus dem Beleuchtungskörper bestrahlt. Der Beleuchtungskörper in Form einer Weißlichtquelle weist eine herkömmliche Glühlampe, Quarzlampe, Leuchtstoffröhre und/oder Leuchtdiode auf. Bei dem durch den Beleuchtungskörper ausgesendeten Licht handelt es sich um Weißlicht, wobei unter Weißlicht ein Licht mit einem Wellenlängenspektrum verstanden werden soll, welches zumindest teilweise im sichtbaren Bereich liegt, welches jedoch kein von einer Laservorrichtung ausgesendetes Licht ist.

Hinsichtlich weiterer Ausbildungen des Verfahrens und einzelner Verfahrensschritte zur Realisierung der Identifikation der Funktion eines biologischen Moleküls im Wege der Inaktivierung, die zum technischen Erfolg der Erfindung beitragen und für die Schutz begehrt wird, wird auf die am 24. November 1998 unter der Anmeldenummer 198 54 195.3 eingereichte deutsche Patentanmeldung desselben Anmelders verwiesen, wobei auf den Offenbarungsgehalt dieser Anmeldung, insbesondere sämtliche Merkmale der Ansprüche 1 - 6, voll inhaltlich Bezug genommen wird.

Weitere Vorteile, Merkmale und Anwendungsmöglichkeiten der Erfindung werden nun unter Bezugnahme auf die beigefügten Zeichnungen anhand von Ausführungsbeispielen detailliert erläutert. Es zeigen:
- Fig. 1: den prinzipiellen Aufbau der erfindungsgemäßen CALI-Vorrichtung;
- Fig. 2: eine Draufsicht eines als Positioniertisch ausgebildeten Probenhalters;
- Fig. 3: eine Seitenansicht des Positioniertisches gemäß Fig. 2;
- Fig. 4: eine mit Peltier-Element ausgebildete Unterlage auf dem Positioniertisch zur Aufnahme von Mikrotiterplatten;
- Fig. 5: eine perspektivische Ansicht eines als Inkubator um den CALI-Positioniertisch gemäß Fig. 2 ausgebildeten Gehäuses;
- Fig. 6: eine Darstellung von Versuchsergebnissen bezüglich einer Inaktivierung von β-Galaktosidase bei Verwendung von MG;
- Fig. 7: Versuchsergebnisse hinsichtlich der Potenz von MG-CALI und von FITC-CALI;
- Fig. 8: Versuchsergebnisse bezüglich der Inaktivierung von β-Galaktosidase bei Verwendung von FITC-unter Licht und unter Dunkelbedingungen;
- Fig. 9: den Einfluß von Tageslicht auf die Inaktivierung von β-Galaktosidase mittels FITC-markiertem Antikörper;
- Fig. 10: den spezifischen Inaktivierungsgrad von β-Galaktosidase mittels FITC-CALI in Abhängigkeit von der Bestrahlungsdauer mit einer diffusen Weißlichtquelle;
- Fig. 11.1 bis 11.3: das Flußbild der in der Steuereinrichtung verwendeten zentralen Systemsoftware; und
- Fig. 12.1 und 12.2: den prinzipiellen Gesamtaufbau der erfindungsgemäßen CALI-Vorrichtung bei Verwendung einer Weißlichtquelle.

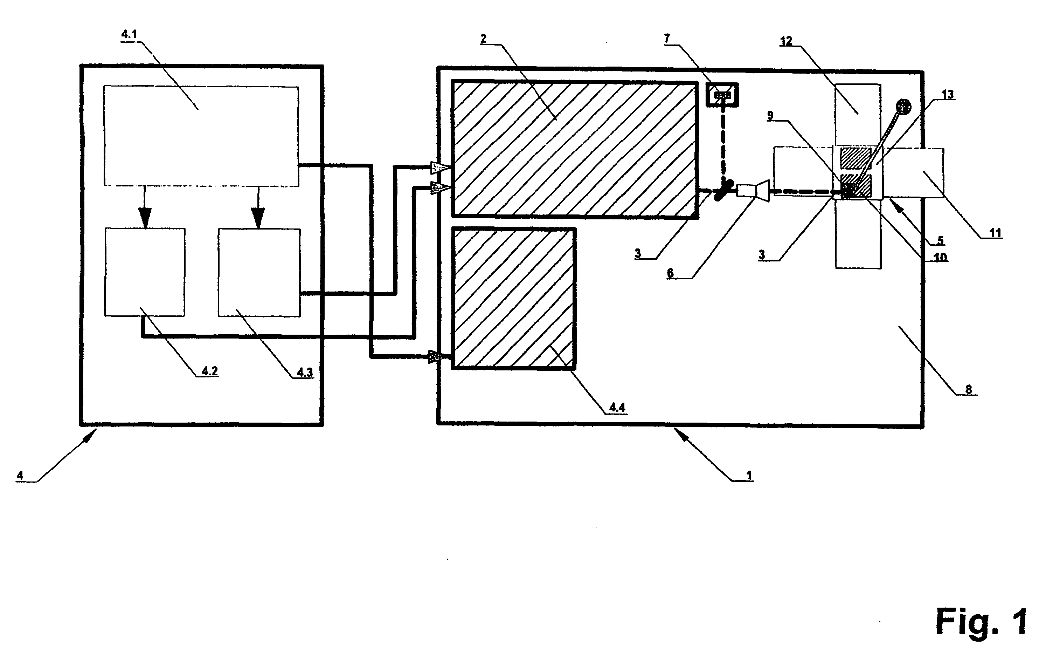

Fig. 1 zeigt den prinzipiellen Aufbau der CALI-Vorrichtung gemäß der Erfindung. Die im Rahmen eines vollautomatisierten Laboruntersuchungsvorganges eingesetzte CALI-Vorrichtung 1 weist eine Laservorrichtung 2 auf, aus welcher ein Laserstrahl 3 austritt. Der Laserstrahl 3 dient der Bestrahlung von Proben zur Identifikation der Funktion von biologischen Molekülen, welche in Kavitäten angeordnet sind, welche sich in Mikrotiterplatten 10 befinden, wobei die Mikrotiterplatten 10 auf einem Probenhalter 5 montiert sind. Der Probenhalter 5 weist einen Positioniertisch 13 in Form eines Kreuztisches mit Verschiebemechanismen 11, 12 auf, so daß die die Mikrotiterplatte 10 tragende Baugruppe entlang der Verschiebemechanismen 11, 12 in die x- und y-Richtung verschiebbar ist. Der Verschiebemechanismus weist zwei mit Verschiebeschienen versehene Bauteile auf: Eine erste, der Bewegung der Mikrotiterplatte 10 in x-Richtung dienende Gleitführung 11 und eine zweite, der Verschiebung der Mikrotiterplatte 10 in die y-Richtung dienende Gleitführung 12. Um eine entsprechende Verschiebung der Mikrotiterplatte 10 in die x-Richtung bzw. die y-Richtung auszuführen, sind Antriebe für die jeweiligen Gleitführungen vorgesehen, so daß entsprechend der Verschiebung in die jeweilige Richtung die in der jeweiligen Mikrotiterplatte 10 vorhandenen mehreren Kavitäten zur Aufnahme der Proben nacheinander bei im wesentlichen feststehendem Laserstrahl 3 der Bestrahlung mittels der Laservorrichtung 2 ausgesetzt werden können. Zur Erhöhung der Flexibilität der CALI-Vorrichtung ist zwischen Austritt des Laserstrahls 3 aus der Laservorrichtung 2 und Eintritt in ein im Bereich der Mikrotiterplatte 10 angeordnetes Prisma 9 eine Strahlmodifikationseinrichtung 6 vorgesehen. Mittels dieser Strahlmodifikationseinrichtung 6 wird der Laserstrahl 3 an die jeweilige Probe angepaßt. Das bedeutet, daß der Laserstrahl 3 in seiner Form an die Probe bzw. die Größe der Kavität in der Mikrotiterplatte 10 und auch hinsichtlich seiner Intensität an die jeweilige Probengröße anpaßbar ist. Des weiteren ist die Strahlmodifikationseinrichtung 6 so ausgebildet, daß der Laserstrahl an die Probenart und/oder auch die Probenanzahl anpaßbar ist. Durch eine zur CALI-Vorrichtung gehörende zentrale Steuereinrichtung 4, welche eine zentrale Systemsoftware 4.1 XCALIbur anwendet, werden sämtliche Bewegungsabläufe, Leistungen und Zustandsbedingungen der automatisierten CALI-Vorrichtung 1 gesteuert bzw. auf bestimmte vorgegebene Werte eingeregelt.

Um eine Reproduzierbarkeit der Untersuchungen durchführen zu können und um die an die jeweiligen Probenart angepaßte Intensität des Laserstrahls 3 einstellen und überwachen zu können, ist am Austritt der Laservorrichtung 2 ein Leistungsmesser 7 vorgesehen, welcher die austretende Leistung des Laserstrahls 3 anzeigt bzw. registriert. Die gesamte Vorrichtung ist auf einem optischen Tisch 8 als Einheit angeordnet.

Als Pumplaser wird ein gepulster Nd:YAG-Infrarot-Laser benutzt, beispielsweise von der Firma Infinity, Coherent, dessen dritte harmonische Wellenlänge von 355 nm verwendet wird, um einen optischen parametrischen Oszillator (Infinity-XPO, Coherent) zu betreiben. Der Pumplaser und der XPO werden jeweils getrennt über zwei benutzerfreundliche Menüprogramme (Steuermodule 4.2, 4.3) über die Steuereinrichtung 4 mit XCALIbur zentral computergesteuert. Pulsenergien von mehr als 30 mJ/Puls sind erzeugbar, wobei die Pulswiederholungsfrequenzen im Bereich von 1 - 30 Hz frei wählbar sind. Die typische Pulslänge liegt bei 3 ns. Die erzeugbaren Wellenlängen des Nutzsignalstrahls reichen von 420 bis 709 nm und die des unbenutzten sog. "Idler"-Strahls von 2300 bis 709 nm.

Um bei einer On-Line-Messung mit Strahlauskopplung die Laserausgabeleistung während des Experiments bzw. der Bestrahlung mittels des Leistungsmessers 7 zuverlässig steuern zu können, werden ca. 2% des aus dem XPO austretenden Strahls mittels einer Auskopplungsoptik (25,4 mm Durchmesser, Coherent, Katalog-Nr. 44-2343) vom Laserstrahl 3 abgetrennt und auf einen thermischen Energiemeßkopf (LMP 10i, Coherent, Katalog-Nr. 33-1173) geleitet. Die auftreffende Energie wird durch einen Energie-/Leistungsanalysator (wie beispielsweise einen Fieldmaster-GSTM, Coherent, Katalog-Nr. 33-0498) angezeigt. Dieser Energie/Leistungsanalysator ist über eine RS-232-Schnittstelle zur Dokumentation der on-line gewonnenen Meßdaten in die "XCALIbur" genannte zentrale Steuersoftware 4. 1 integriert.

Für die Laserstrahl-Durchmesserregulierung (zoom beam expander) wird ein Präzisions-Laseraufweiter (Coherent, Auburn Group, Katalog-Nr. 31-2505) mit einem Aufweitungsfaktor von 1 mal bis 8 mal eingesetzt. Durch eine Antireflexbeschichtung ist die Lichttransmission bei 630 nm, d.h. in der Nähe der für die CA-LI-Untersuchungen mit MG benutzten Wellenlänge, optimiert (> 94%).

Vor dem Auftreffen des Laserstrahls 3 auf die jeweilige Probe ist der Laserstrahl 3 durch ein Rechtwinkelprisma 9 mit 40 mm Seitenlänge (Produkt-Nr. 01PRA029, Melles Griot, Zevenaar, NL) geführt, dessen Hypotenuse eine Silberbeschichtung zur Totalreflexion und dessen Kathete mit Breitband-Antireflexbeschichtung Hebbar™ (415 - 700 nm, Produkt-Nr./078) versehen sind. Damit wird der Laserstrahl 3 aus der Horizontalen um 90° in die Vertikale in Richtung auf den Positioniertisch 13, d.h. die Kavität mit der Probe in der Mikrotiterplatte 10, umgelenkt. Um den Probendurchsatz bei der Laserbestrahlung zu erhöhen, wird ein Breitbandstrahlteiler (25,4 mm Durchmesser, 50 : 50 Strahlteilung bei 420 bis 700 nm, Coherent, Katalog-Nr. 44-2168) vor dem Rechtwinkelprisma 9 eingeführt, welcher die Hälfte des eingehenden Laserstrahls 3 bzw. dessen Energie im 90°-Winkel zum durchgelassenen Strahlanteil auskoppelt. Je nach für eine jeweilige Probe zur Gewährleistung von CALI-Inaktivierung benötigter Energie, und zwar in Abhängigkeit vom verwendeten Farbstoff, ist eine 3-fache, 4-fache oder gegebenenfalls 8-fache Strahlteilung durch serielles Einfügen von Strahlteilern mit einem jeweiligen geeigneten Auskopplungsverhältnis vorgesehen. Dabei ist eine entsprechende Kombination von 1, 2, 3, 5 und 9:1-Auskopplern von Coherent möglich.

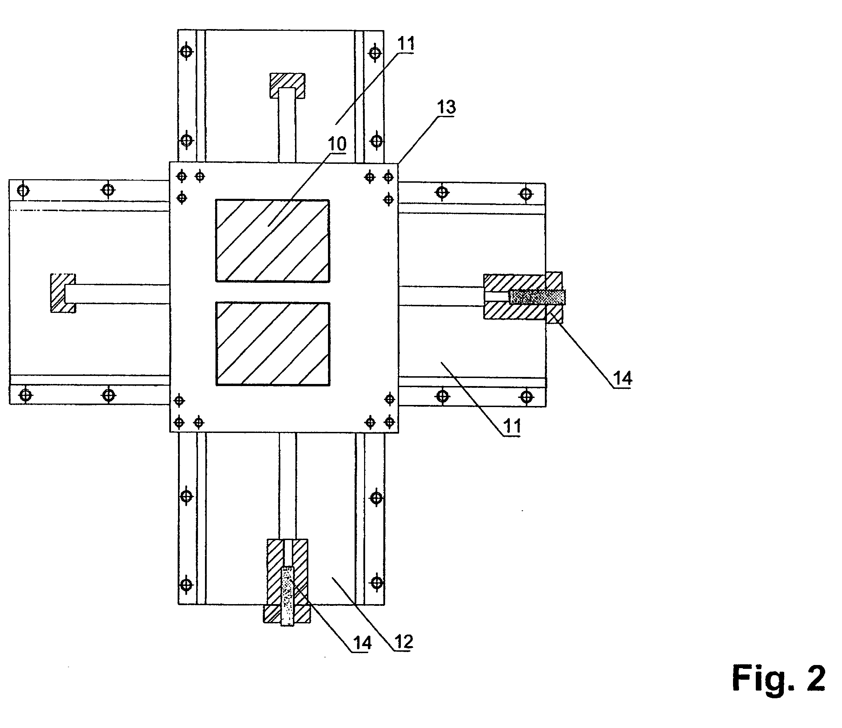

In Fig. 2 ist die Draufsicht auf den als Kreuztisch ausgebildeten Probenhalter 5 zur Aufnahme der Mikrotiterplatte 10 dargestellt. Der Probenhalter 5 weist eine Gleitführung 12 auf, auf welcher der eigentliche, die Mikrotiterplatte 10 tragende Positioniertisch 13 an einer entsprechenden Führung angebracht ist. Der Antrieb zur Bewegung des Tisches in die y-Richtung erfolgt mittels Schrittmotor 14.

Die Gleitführung 12 ihrerseits ist auf einer weiteren, rechtwinklig dazu angeordneten, die Bewegung in die x-Richtung realisierenden Gleitführung 11 angeordnet. Der Antrieb der Gleitführung 12 zu deren Bewegung auf der Gleitführung 11 in die x-Richtung erfolgt ebenfalls mit einem Schrittmotor 14.

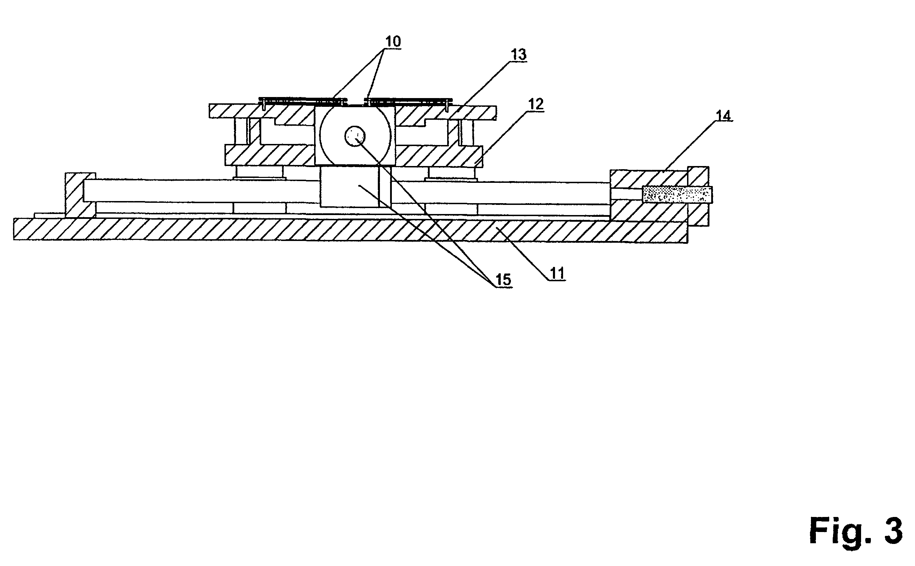

In Fig. 3 ist eine Schnitt-Seitenansicht des Positioniertisches 13 dargestellt. Daraus ist ersichtlich, daß die Gleitführungen 11, 12 im Huckepack-Prinzip übereinander angeordnet und relativ zueinander bewegbar sind, so daß durch die Bewegungen in die x-Richtung und die y-Richtung die auf dem eigentlichen Positioniertisch 13 angeordneten Mikrotiterplatte 10 innerhalb eines Flächenbereiches zuverlässig bewegbar sind. Eine hohe Genauigkeit der Bewegung der jeweiligen Gleitführungen 11, 12 und damit der Mikrotiterplatte 10 wird durch Kugelspindeln 15 realisiert.

Die Kugelspindeln 15 sind in Antriebswirkverbindung mit den Schrittmotoren 14, welche als 3-Phasen-Schrittmotoren, wie beispielsweise Schrittmotoren der Firma Berger, VDRM 368, ausgebildet sind. Die Steuerung dieser Schrittmotoren 14 erfolgt über ein CNC-Steuermodul 4.4 in Form einer industriellen CNC-Standard-Steuervorrichtung, wie z.B. SM300, SM Electronics GmbH. Dieses CNC-Steuermodul 4.4 arbeitet im Befehlsmodus und ist über entsprechende elektronische Verbindungen direkt mit der Steuereinrichtung 4 gekoppelt bzw. ist ein Teil davon.

Die auf dem Positioniertisch 13 angeordneten zwei Mikrotiterplatten 10 mit den Kavitäten zur Aufnahme der Proben weisen in an sich bekannter Weise jeweils 96 Kavitäten auf, so daß es möglich ist, 192 Proben nacheinander zu untersuchen bzw. zu bestrahlen, ohne daß es erforderlich ist, daß eine Bedienperson eingreift. Selbstverständlich ist es auch möglich, andere Mikrotiterplatten auf dem Positioniertisch 13 anzuordnen, welche eine von 96 verschiedene Anzahl von Kavitäten aufweisen.

Die Verbindung des CNC-Steuermoduls 4.4 für die 3-Phasen-Schrittmotoren 14 der Kugelspindel-Antriebe ist über ein serielles Kabel mit dem RS-232-Port der Steuereinrichtung 4 verbunden.

Der Einsatz von Kugelspindeln 15 ermöglicht neben der exakten Einstellung eine Wiederholbarkeitsgenauigkeit von mindestens 0,1 mm. Dies ist notwendig, damit der Laserstrahl 3 stets die Mitte der Mikrotiterplatten-Kavität trifft und dadurch die Probe vollständig bestrahlt wird und Strahlstreuung an den Rändern der Kavität vermieden wird.

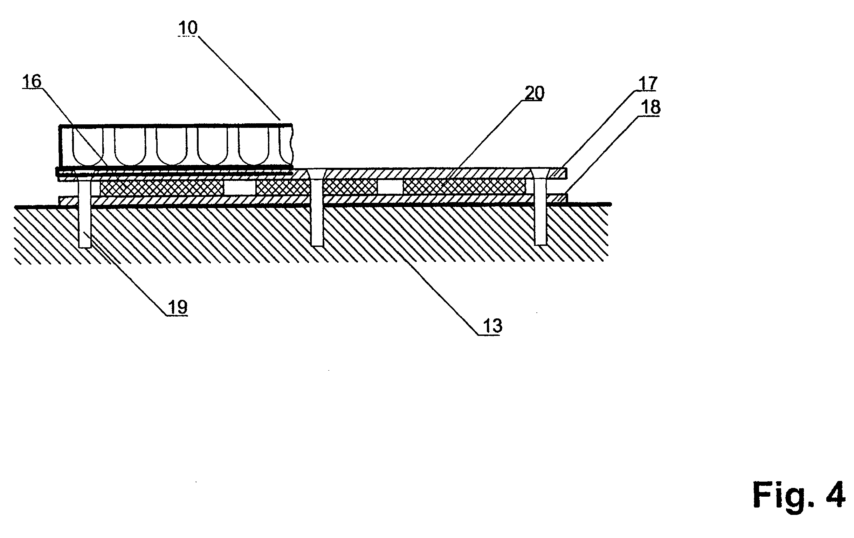

In Fig. 4 ist der prinzipielle Aufbau eines Trägers 16 bzw. Rezeptors für Mikrotiterplatten 10 dargestellt. Der Träger 16 weist eine Sandwich-Struktur mit 6 Peltier-Elementen 20, vorzugsweise von CONRAD ELECTRONIC, 7105, auf, welche zwischen zwei schwarz anodisierten Stahlplatten 17, 18 montiert sind. Die gesamte Sandwich-Struktur ist mittels Schrauben 19 mit dem Positioniertisch 13 verbunden. Es ist jedoch auch möglich, eine anderweitige Verbindung, beispielsweise durch Magnete zu realisieren, um eine Automatisierung des gesamten Verfahrensablaufs zu ermöglichen bzw. zu erleichtern. Die Mikrotiterplatten 10 sollten bei einer moderaten Temperatur mit Hilfe dieser Peltier-Elemente 20 über einen Bereich von etwa 15°C bis 45°C unabhängig gehalten werden, wobei die Temperatur über nicht dargestellte externe oder interne Sensoren überwacht wird.

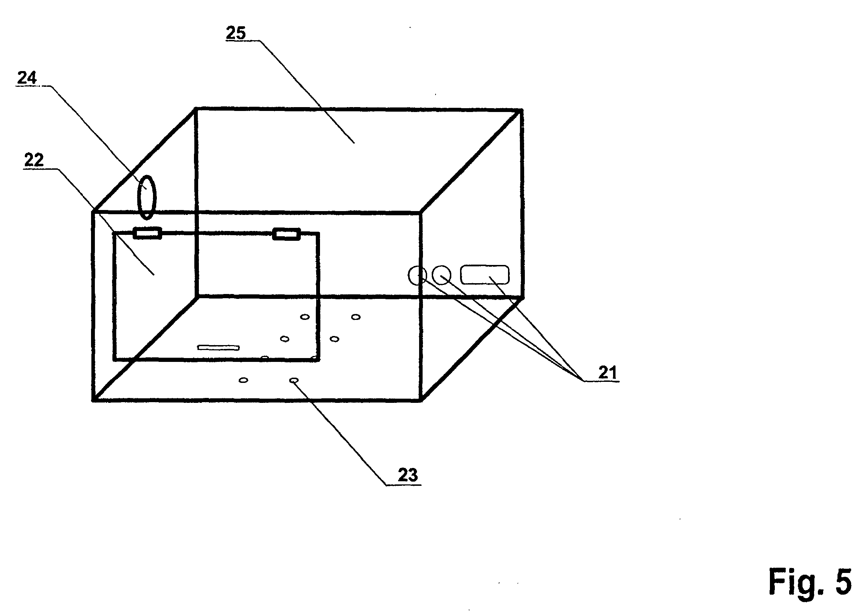

Der Einfachheit der Darstellung wegen ist in Verbindung mit den Fig. 1 bis 4 nicht beschrieben worden, daß der Positioniertisch 13 mit den Mikrotiterplatten 10 (CALI-Kreuztisch) von einem Gehäuse (nicht dargestellt in Fig. 4) umgeben ist, welches aufgrund der Tatsache, daß innerhalb des Gehäuses eine definierte Atmosphäre für jeweilige Proben aufrechterhalten bzw. eingestellt wird, auch als Inkubator bezeichnet wird. Dieser Inkubator 25 ist in prinzipieller perspektivischer Ansicht in Fig. 5 dargestellt. Der Inkubator 25 weist in Seitenwänden einen Durchbruch 24 fiir die Bestrahlungssoptik sowie Durchbrüche 21 für Versorgungsleitungen, wie z.B. elektrische Versorgungsleitungen, Luftleitungen, Gasleitungen, etc., auf. Des weiteren weist der Inkubator 25 eine verschließbare größere Öffnung in Form einer Klappe 22 auf, durch welche beispielsweise die Mikrotiterplatte 10 in den Inkubator 25 eingebracht werden kann. Dies kann beispielsweise mit einem Roboterarm geschehen. Die verschließbare Klappe 22 ist im verschlossenen Zustand je nach Zustandsbedingungen im Inneren des Inkubators 25 gasdicht bzw. temperaturisoliert abgedichtet. Am Boden des Inkubators 25 sind weitere Öffnungen 23 in Form von Befestigungsöffnungen vorgesehen, welche der Befestigung des Inkubators 25 auf dem optischen Tisch 8 dienen.

Nicht dargestellt sind entsprechende Ventile in den Versorgungsleitungen, mittels welcher eine Einstellung der atmosphärischen Zustandsbedingungen im Inneren des Inkubators 25 erfolgt.

Die in Fig. 1 dargestellte, mit der zentralen Systemsoftware 4.1 arbeitende Steuereinrichtung 4 ist so ausgebildet, daß die Ventile (nicht dargestellt) der Versorgungsleitungen ansteuerbar und betätigbar sind, so daß die atmosphärischen Zustandsbedingungen im Inneren des Inkubators 25 einstellbar sind. Gleichermaßen ist es möglich, die Öffnungsklappe 22 mit einem Antrieb zu versehen, welcher über die zentrale Steuereinrichtung 4 betätigbar ist, so daß auch die Beschickung des Inkubators 25 mit auf insbesondere der Mikrotiterplatte 10 angeordneten Proben im automatisierten CALI-Vorgang erfolgen kann.

Die zentrale Steuersoftware 4.1 (XCALIbur) bildet das Nutzer-Interface für sämtliche Bewegungs- und Steuervorgänge der gesamten CALI-Vorrichtung 1. Während die Steuerung des Positioniertisches 13 über eine serielle Schnittstelle (RS-232) der System-Steuereinrichtung 4, welche ein PC ist, erfolgt, wird die Steuerung der Laservorrichtung 2 mittels Windows DDE-Server durchgeführt, welche in die Software für das Lasersteuermodul 4.2 und die XPO 4.3 integriert sind, wodurch ein fehlerhafter Betrieb der Laservorrichtung 2 durch den Nutzer ausgeschlossen ist. Die XCALIbur-Steuersoftware 4.1 ermöglicht auch die Bedienung des Inkubators 25 einschließlich der darin einzustellenden atmosphärischen Bedingungen.

Ein Nutzer kann bei Verwendung der XCALIbur-Software jede einzelne Kavität und damit jede einzelne Probe in der Mikrotiterplatte 10 anwählen und sämtliche Laserparameter bzw. Lichtstrahlparameter der in der jeweiligen Kavität angeordneten Probe differenziert zuordnen. Die Reihenfolge der Bestrahlung wird ebenfalls einfach per Mausklick durch den Nutzer vorgegeben.

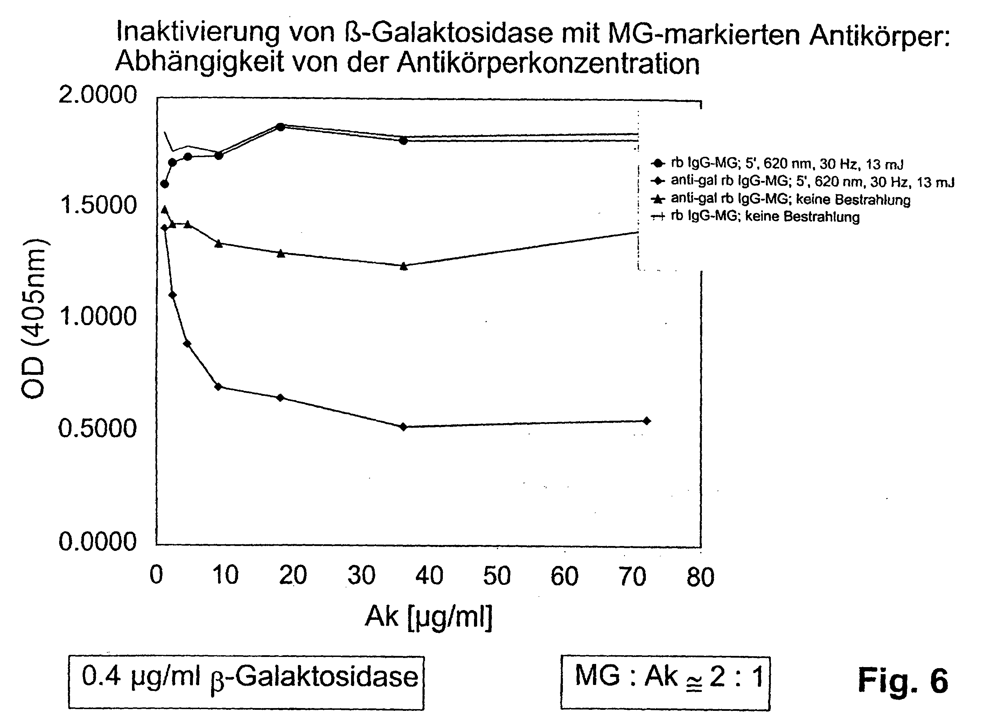

In den Figuren 6 bis 10 sind die wichtigsten Ergebnisse eines experimentellen Protokolls zur Probenvorbereitung und Ausführung von in vitro CALI am Beispiel von β-Galaktosidase zusammengefaßt. Zur Kopplung des Farbstoffes an das Effektor-Molekül (Antikörper) wurde Malachitgrün-Isothiocyanat (MG) oder Fluoreszein-isothiocyanat (FITC) verwendet. Als spezifischer Antikörper wurde gereinigtes polyklonales Kaninchen-anti-Galaktosidase IgG-Antiserum verwendet. Als Negativkontrolle diente gereinigtes IgG von nichtimmunisierten Kaninchen. Die Antikörper wurden zuerst gegen PBS, pH 7,4 dialysiert, und zwar 3 Stunden mit einem Molekulargewichtsausschluß von 3500 Da. Danach wurden 600 µg Antikörper zusammen mit der gleichen Menge Rinderserumalbumin (RSA) im Falle von MG und ca. 400 µg Antikörper ohne Rinderserumalbumin im Falle von FITC zur Kopplung eingesetzt. MG und FITC wurden in einer Konzentration von 10 mg/ml in Dimethylsulfoxid gelöst. Das Koppeln fand für MG in 600 µl 0,5 M NaHCO₃, pH 9,5 statt bei maximal 10% Antikörperbeitrag zum Volumen. MG wurde in kleine Aliquots auf Eis zum Antikörper zugegeben, und zwar 5 mal 1µl nach je 1 min, nach weiteren 5 min 2,5 µl und nach 2,5 min nochmals 2,5 µl, nach 5 min 5µl und nach weiteren 10 min nochmals 5 µl, abschließend 30 min Inkubation, d.h. insgesamt 20 µl MG mit ca. 1 Stunde Kopplungszeit. Anschließend wurde nichtgekoppeltes MG mittels Gelfiltration abgetrennt. Dies erfolgte mittels einer nicht dargestellten PD 10-Säule mit Sephadex G25 Matrix, beispielsweise von der Firma Pharmacia. Dazu wurde die Säule zuerst in PBS, pH 7,4 (4 mM KH₂PO₄, 16 mM Na₂HPO₄, 115 mM NaCl) equilibriert. Die Kopplungsreaktion wurde mit PBS auf 1ml aufgefüllt und auf die Säule geladen. Danach wurde mit 2 ml PBS gewaschen und anschließend der MG-gekoppelte Antikörper in 1,5 ml PBS eluiert. Das Kopplungsverhältnis der Probe durch Absorptionsmessung bei 620 nm (MG-Anteil) und Proteinkonzentrationsbestimmung mittels Bradford Assay (Antikörperanteil) bestimmt. Für die Kopplung mit FITC wurde das Protokoll des Herstellers befolgt. 400 µg Antikörper in 180 ml PBS wurden mit 40 µl 0,5 M NaHCO₃, pH 9,5 versetzt, wobei ein in Abhängigkeit von der Proteinkonzentration errechnetes Volumen von 3,2 µl FITC zugegeben wurde. Die Kopplung fand lichtgeschützt während 1 Stunde bei Raumtemperatur statt, wobei die Kopplungsreaktion ebenfalls über eine Gelfiltrationssäule gereinigt wurde. Das Kopplungsverhältnis der Probe wurde durch Absorptionsmessung bei 494 nm (FITC-Anteil) und 280 nm (Proteinkonzentrationsbestimmung) unter Berücksichtigung entsprechender Korrekturfaktoren (nach der Formel von Molecular Probes) bestimmt.

Die Vorbereitung der Proben und die Laser- bzw. Lichtbestrahlung erfolgte wie folgt:

Die Proben wurden zuerst in Eppendorf-Reaktionsgefäßen angesetzt, und zwar 15 µl Gesamtvolumen pro Probe, und 1 Stunde lang auf Eis vorinkubiert, um eine Antikörper-Antigenbindung zuzulassen. Zu einer konstanten Menge gereinigter β-Galaktosidase (0,67 U/ml, 1,3 µg/ml; Reinheitsgrad X, Sigma) wurde die gewünschte Menge Farbstoff-gekoppelter Antikörper zugegeben, und zwar zwischen 3,75 und 240 µg/ml, wobei mit ca. 60 µg/ml gewöhnlich die nahezu maximale Inaktivierung erzielt wurde, was einem molaren Verhältnis von Enzym : Antikörper von etwa 1:45 entspricht. Neben der Zugabe der gewünschten Menge an Farbstoff-gekoppeltem Antikörper wurden 250 µg/ml RSA als Träger zugegeben, um eine unspezifische Absorption des Enzyms an dem Reaktionsgefäß zu verhindern. Danach wurde mit 1 x PBS, pH 7,4 auf 15µl pro Probe aufgefüllt. Nach der Vorinkubation wurde das Probenvolumen mit 1 x PBS auf 50 µl aufgefüllt (Verdünnungsfaktor 3,3 x) und die RSA-Konzentration im Ansatz wieder auf 250 µg/ml gebracht. Die Proben wurden als unabhängige Dreifachbestimmungen pro Behandlungstyp (Probentyp) in die Kavitäten einer Mikrotiterplatte mit 96 Kavitäten, einer sog. Flachboden-Mikrotiterplatte, überführt und einer Laserbestrahlung unter den gewünschten Bedingungen ausgesetzt.

Für das CALI-Verfahren unter Verwendung einer Laservorrichtung 2 wurde der Laser üblicherweise mit ca. 270 mJ/Puls Infrarotenergie mit einer Wellenlänge von 1064 nm betrieben, was einer Bestrahlungsenergie von ca. 13 mJ/Puls bei 620 nm und 15 mJ/Puls bei 494 nm entsprach, und zwar gemessen mit dem Fieldmaster-Energiemeßgerät über ca. 2% Auskopplung des Strahls. Der Laserstrahl 3 hatte einen Durchmesser von ca. 5 mm und überstrich die Gesamtfläche einer Kavität. Die Bestrahlungsdauer lag gewöhnlich zwischen 1 bis 5 min.

Bei Anwendung von FITC für CALI in Kombination mit diffusem Licht wurde eine Weißlichtglühlampe verwendet und eine gewöhnliche Schreibtisch-Schirmlampe mit 18 cm Durchmesser senkrecht über den Proben in ca. 20 cm Abstand aufgestellt, und die in einer Mikrotiterplatte mit 96 Kavitäten auf Eis befindlichen Proben 0 bis 60 min lang bestrahlt.

Nach erfolgter Bestrahlung wurde der Inaktivierungsgrad bestimmt. Der Inaktivierungsgrad von β-Galaktosidase wurde über das ONPG (Orthonitrophenylgalactosidase)-Nachweisverfahren bestimmt. Dazu wurden zu den 50 µl Probe 50 µl Reaktionspuffer A gegeben (100 mM NaH₂PO₄, pH 7,5; 10 mM KCI, 1mM MgSO₄) sowie 50 µl ONPG-Lösung (4 mg/ml in 100 mM NaH₂PO₄, pH 7,2) gegeben. Dieses Gemisch wurde bei 37°C eine definierte Zeit lang (zumeist 30 min) inkubiert und die Reaktion danach mit 90 µl 1 M Na₂CO₃ gestoppt. Der Umsetzungsgrad des ONPG-Substrates (Gelbfärbung) wurde in den einzelnen Proben bei 405 nm in einem ebenfalls nicht direkt beschriebenen Mikrotiterplatten-Absorptionsspektrophotometer (Tecan) bestimmt.

Die in den Fig. 6 bis 10 dargestellten Ergebnisse lassen sich wie folgt interpretieren. Um die Effektivität des neuen CALI-Systems mittels des eingesetzten Nd:YAG-Lasers zu testen, wurde die CALI-Inaktivierung am Beispiel von ß-Galaktosidase durchgeführt. Die bereits als CALI-tauglich beschriebenen verschiedenen Farbstoffe MG und FITC wurden eingesetzt. Die Bestrahlungsbedingungen, die Bestrahlungsabfolge und die Bestrahlungsintensität wurde mittels der XCALIbur-Software programmiert, wobei die Proben damit automatisch abgearbeitet wurden.

In Fig. 6 ist eine Titration der Konzentration der verwendeten MG-gekoppelten Antikörper gezeigt (ONPG-Assay). Alle Proben wurden in unabhängigen Dreifachansätzen behandelt. Der Standardfehler mit dem neuen automatisierten CALI-System lag generell bei < 10%. Die halbmaximale Inaktivierung von ß-Galaktosidase war unter den gegebenen Bedingungen, welche bei 5 min, 620 nm, 30 Hz, 13 mJ/Puls lagen, bei ca. 5 µg/ml spezifischem anti-β-Gal-Antikörper erreicht (ca. 10-facher Antikörperüberschuß). Die Sättigung des Inaktivierungseffektes, welcher einem Wert von 60% spezifischer CALI-vermittelter Inaktivierung entspricht, trat bei 35 µg/ml auf (ca. 70-facher Antikörperüberschuß). Das verwendete polyklonale anti-β-Gal-Serum zeigte bereits ohne Laserbestrahlung einen inhibitorischen Effekt, welcher jedoch durch Laserbestrahlung wesentlich verstärkt wurde. Das verwendete Kontroll-IgG-Serum bewirkte mit und ohne Laserbestrahlung keinen inhibitorischen Effekt.

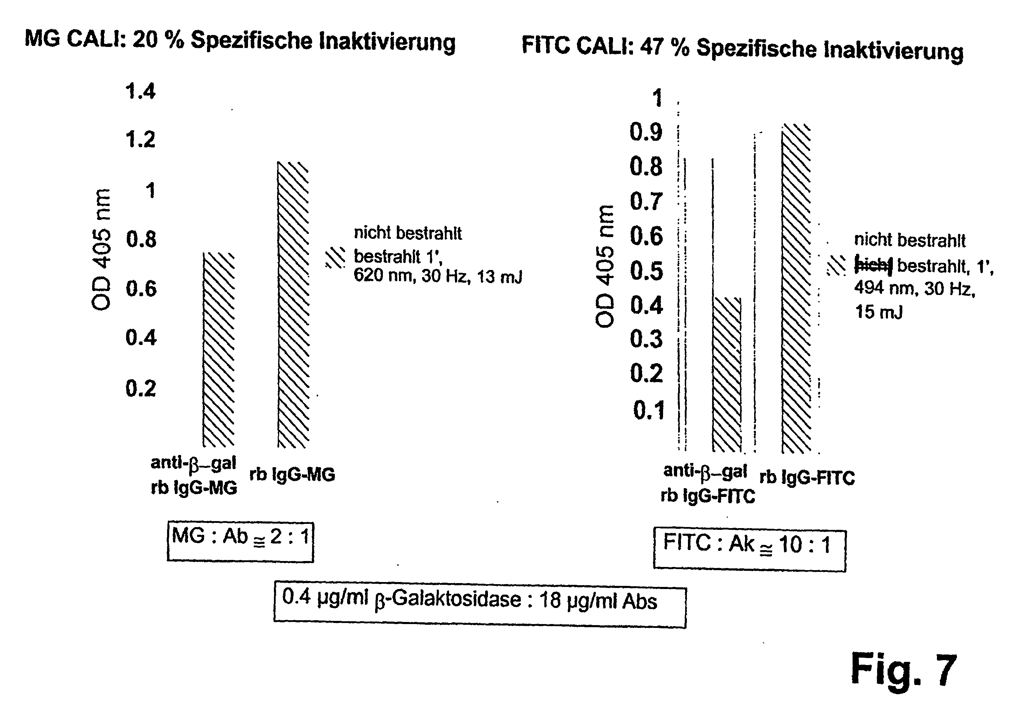

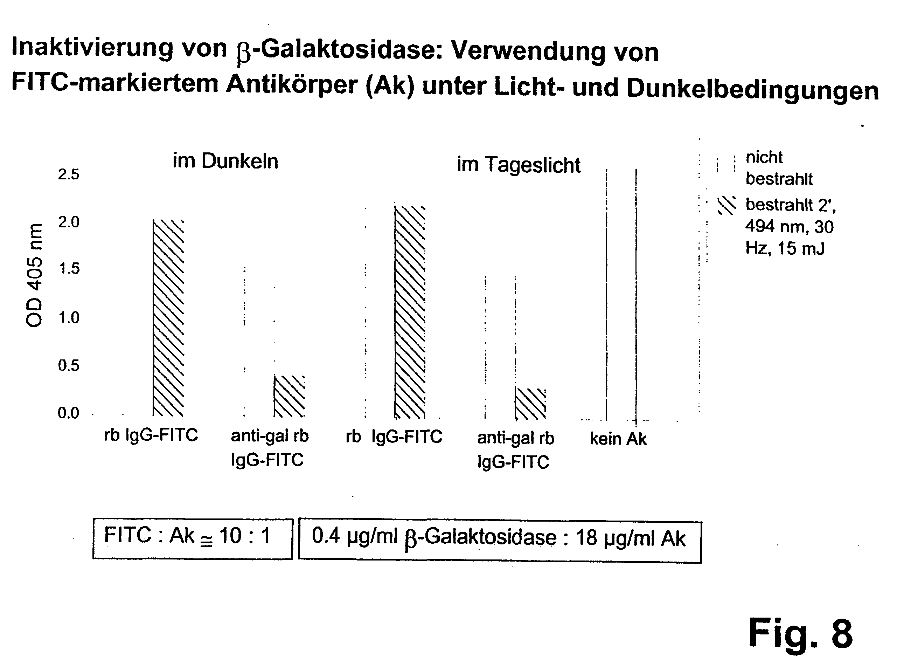

Die Verwendung von FITC, welches eine ca. 5-fach bessere Kopplung zum Antikörper aufwies als MG (10:1 FITC gegenüber 2:1 MG), bewirkte eine schnellere und stärkere CALI-vermittelte Inaktivierung als MG, wie aus Fig. 7 und 8 ersichtlich ist. Nach 1 min Bestrahlung waren 47 % Inaktivierung mit FITC gegenüber 20 % mit MG erreicht (Fig. 7). Nach 2 min Laserbestrahlung mit 494 nm bei 30 Hz und 15 mJ/Puls waren 80% der β-Galaktosidaseaktivität (ONPG-Assay) inaktiviert, und zwar bei 45-fachem Antikörperüberschuß (Fig. 8). Dieses Experiment wurde soweit als möglich im Dunkeln durchgeführt. Bei Durchführen des gleichen Experiments unter Einfluß von Tageslicht zeigte sich bereits ohne Laserbestrahlung eine signifikante Inaktivierung von β-Galaktosidase in Gegenwart des spezifischen, FITC-gekoppelten Antikörpers (siehe Fig. 8).

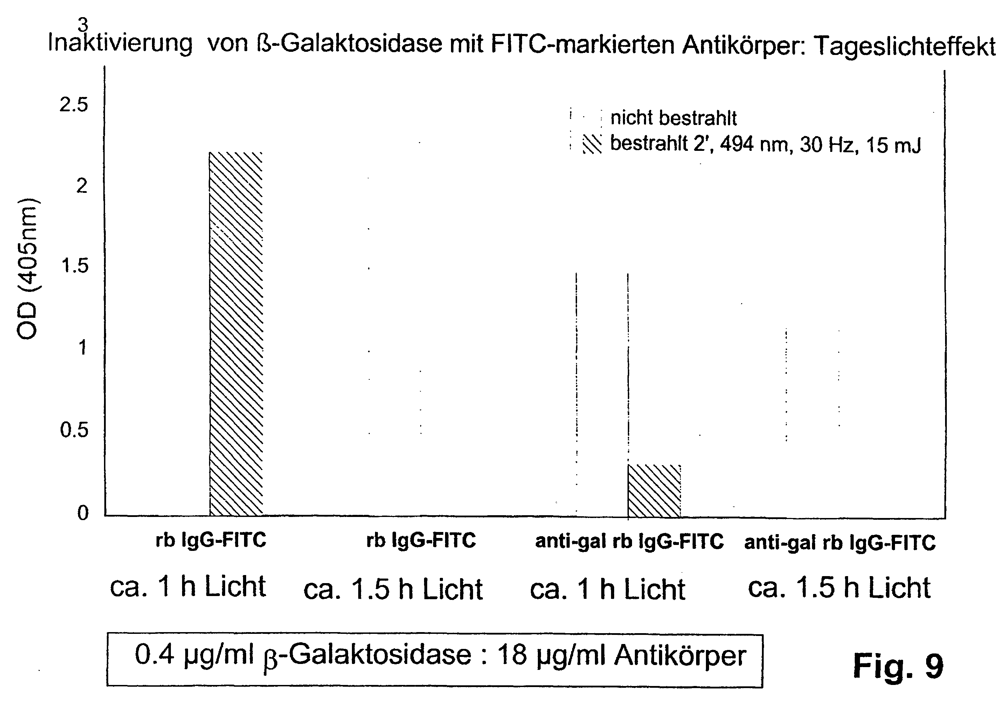

In einem weiteren Experiment, bei welchem der Einfluß der Dauer der Einwirkung von Tageslicht bestimmt wurde, zeigte sich, daß mit zunehmender Inkubationsdauer bei Licht der Laser-unabhängige Inaktivierungsgrad stieg, wie aus Fig. 9 ersichtlich (vergl. dazu die Inaktivierung nach 1 und nach 1,5 Stunden).

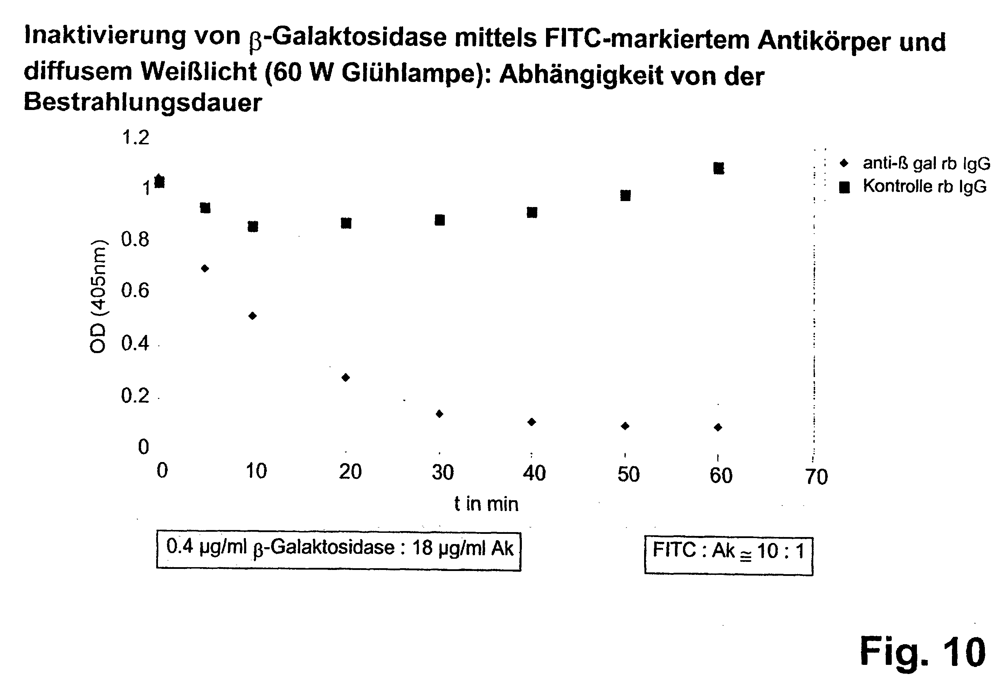

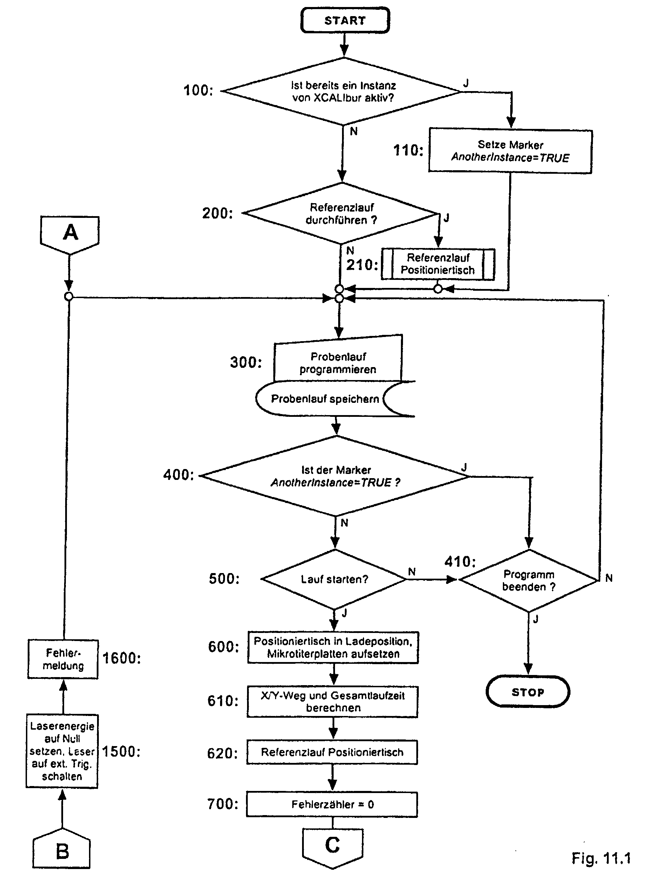

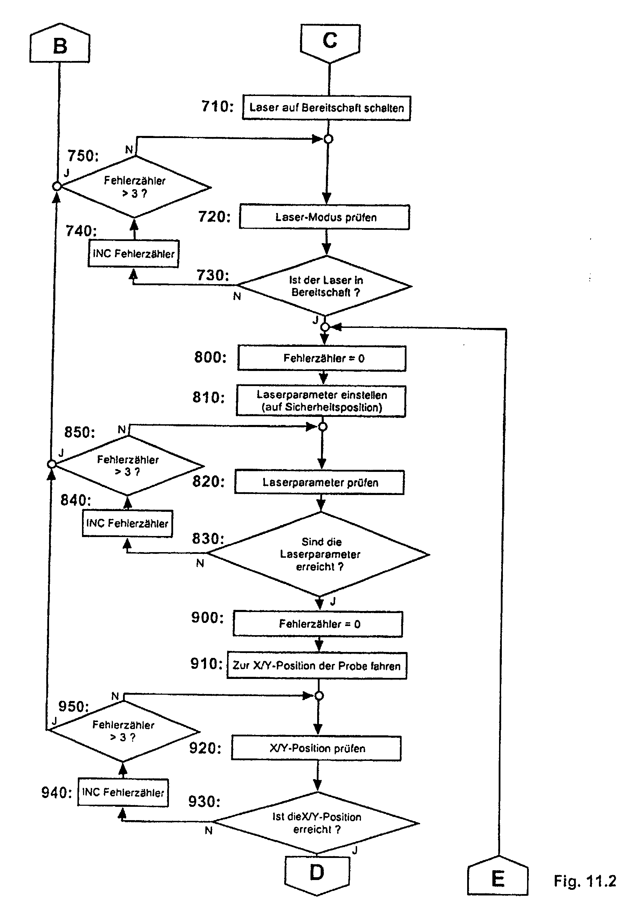

Um diesen FITC-abhängigen Effekt deutlicher herauszuarbeiten, wurde eine Lichtquelle auf der Basis von Weißlicht einer definierten Stärke (Glühlampe mit 60 W Leistung) eingesetzt. Dabei wurde der spezifische Inaktivierungsgrad von β-Galaktosidase in Abhängigkeit von der Bestrahlungsdauer bestimmt. Dies ist in Fig. 10 dargestellt. Aus Fig. 10 ist ersichtlich, daß nach ca. 10 bis 15 min eine halbmaximale Inaktivierung erreicht wurde, und nach ca. 30 min eine Sättigung des Inaktivierungseffektes eintrat.

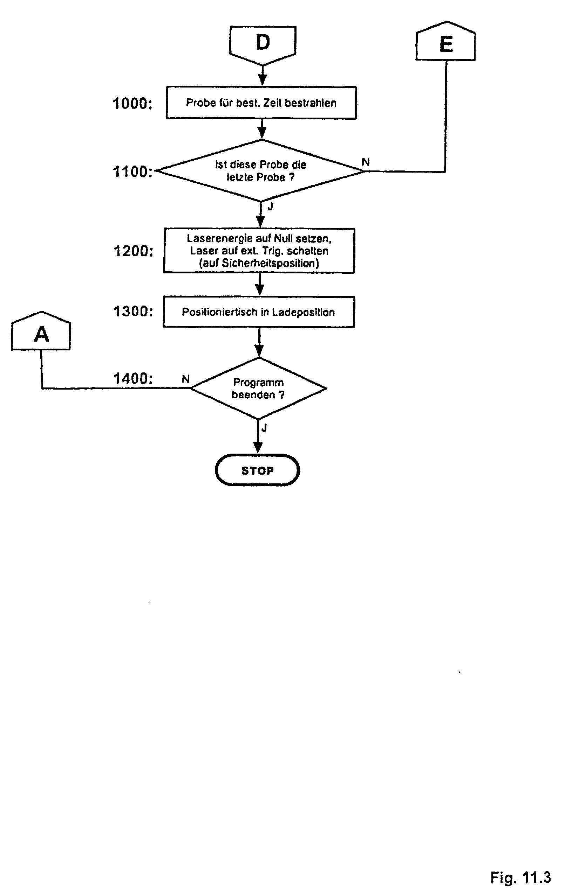

In den Fig. 11.1 bis 11.3 ist das Flußdiagramm XCALIbur beschrieben.
a) Fig. 11.1
   Schritt 100:
      Nach dem Start des Programms wird zunächst geprüft, ob bereits auf diesem Rechner eine Instanz des XCALIbur-Programmes aktiv ist. Sollte dies der Fall sein, wird ein entsprechender Marker (AnotherInstance) gesetzt, der Einfluß auf die weitere Programmausführung hat. Dieses Vorgehen ist notwendig, um sicherzustellen, daß nur eine Instanz des XCALIbur-Programmes die Kontrolle über den Laser und den Positioniertisch hat.
   Schritt 200:
      Wird nur ausgeführt, wenn das gestartete Programm die erste Instanz auf diesem Computer ist. Dem Nutzer wird angeboten, einen Referenzlauf des Positioniertisches zu dessen Kalibrierung durchzuführen. Sollte der Nutzer zustimmen, wird der Referenzlauf durchgeführt (Schritt 210).
   Schritt 300:
      Der Nutzer befindet sich nun in der Eingabemaske, in der die Definition der einzelnen Probenpositionen erfolgt. Die Reihenfolge, in der der Nutzer die Proben definiert, bestimmt die Reihenfolge der späteren Laserbestrahlung. Der Nutzer hat die Möglichkeit, die Definition für den Bestrahlungslauf für spätere oder sofortige Nutzung abzuspeichern. Es sind die gängigen Editierfunktionen wie Kopieren, Einsetzen und Löschen verfügbar.
   Schritt 400:
      Nachdem die Definition des Bestrahlungslaufes abgeschlossen ist, wird der Zustand des Markers AnotherInstance ausgewertet. Im Zustand "TRUE" wird geprüft, ob der Nutzer das Programm beenden möchte (Schritt 410) oder ob er weitere Läufe definieren möchte (zurück zu Schritt 300). Es ist somit möglich, mehrere Bestrahlungsläufe im voraus zu programmieren, auch wenn auf dem Computer bereits eine andere Instanz des Programmes einen aktuellen Bestrahlungslauf durchführt.
   Schritt 500:
      Sollte der Zustand des Markers AnotherInstance nicht den Zustand "TRUE" haben, kann der Nutzer den Bestrahlungslauf starten, weitere Läufe definieren oder auch das Programm beenden.
   Schritt 600:
      Sollte der Nutzer sich in Schritt 500 für den Start des Laufes entschieden haben, wird der Positioniertisch in die sogenannte Ladeposition am vorderen Rand gefahren. In dieser Position ist es sehr einfach möglich, die Mikrotiterplatten auf den entsprechenden Haltern am Positioniertisch zu befestigen, ohne dabei Teile der strahlablenkenden Optik (Strahlmodifikationseinrichtung) zu berühren.
   Schritt 610:
      Es werden der optimale Weg zur Bestrahlung der Proben sowie die Gesamtlaufzeit berechnet.
   Schritt 620:
      Es wird ein Befehl an das CNC-Steuermodul 4.4 ausgegeben, gemäß welchem mittels dem Positioniertisch nacheinander in x- und in y-Richtung Kalibrierläufe durchführbar sind.
   Schritt 700:
      Ein Fehlerzähler wird auf Null gesetzt.
b) Fig. 11.2
   Schritt 710:
      Über die DDE-Schnittstelle der Coherent-Laser-Steuersoftware wird der Laser in den Standby-Modus geschaltet.
   Schritt 720/730/740/750:
      Aus Sicherheitsgründen wird jetzt geprüft, ob der Laser sich auch tatsächlich im Standby-Modus befindet. Sollte dies nicht der Fall sein, wird der Fehlerzähler inkrementiert. Sollte der Fehlerzähler den Wert 3 überschreiten (entspricht drei erfolglosen Versuchen, den Laser in den Standby-Modus zu schalten), wird die Laserenergie auf Null gesetzt (Schritt 1500) und eine Fehlermeldung ausgegeben (Schritt 1600). Danach hat der Nutzer die Möglichkeit, das Problem zu beheben und den Lauf neu zu starten oder das Programm zu beenden.
   Schritt 800:
      Ein Fehlerzähler wird auf Null gesetzt.
   Schritt 810:
      Die Laserparameter (Leistung, Wellenlänge, etc.) für die erste Probe werden auf einer Sicherheitsposition (außerhalb der Bestrahlungsfläche) eingestellt. Das Einstellen des Lasers erfolgt ausschließlich über die DDE-Schnittstelle der Coherent-Laser-Software, so daß Fehlsteuerungen des Lasers durch falsche Parameter ausgeschlossen sind, da diese von der Coherrent-Software abgefangen werden.
   Schritt 820/830/840/850:
      Es wird geprüft, ob die eingestellten Laserparameter erreicht wurden. Sollte dies nicht der Fall sein, wird der Fehlerzähler inkrementiert und das Überprüfen der Laserparameter fortgesetzt. Wenn die Parameter nach drei Versuchen nicht erreicht wurden, wird die Laserenergie auf Null gesetzt (Schritt 1500) und ein Fehlermeldung ausgegeben (Schritt 1600). Danach hat der Nutzer die Möglichkeit, das Problem zu beheben und den Lauf neu zu starten oder das Programm zu beenden.
   Schritt 900:
      Ein Fehlerzähler wird auf Null gesetzt.
   Schritt 910:
      Der Positioniertisch wird auf die Position der zu bestrahlenden Probe gefahren.
   Schritt 920/930/940/950:
      Es wird geprüft, ober der Positioniertisch die entsprechende Position erreicht hat. Sollte dies nicht der Fall sein, wird der Fehlerzähler inkrementiert und erneut in die Position des Positioniertisches abgefragt. Nach drei erfolglosen Versuchen wird die Laserenergie auf Null gesetzt (Schritt 1500) und eine Fehlermeldung ausgegeben (Schritt 1600). Danach hat der Nutzer die Möglichkeit, das Problem zu beheben und den Lauf (oder einen anderen) neu zu starten oder das Programm zu beenden.
c) Fig. 11.3
   Schritt 1000:
      Die Probe wird mit den eingestellten Laserparametern und der programmierten Strahlungsdauer bestrahlt.
   Schritt 1100:
      Es wird geprüft, ob weitere Proben zur Bestrahlung vorgesehen sind. Sollte dies der Fall sein, wird zu Schritt 800 zurückgekehrt.
   Schritt 1200:
      Die Laserenergie wird auf Null gesetzt, und der Laser aus Sicherheitsgründen auf "External Triggering" geschaltet. Da kein externer Trigger anliegt, kann der Laser auch nicht versehentlich wieder eingeschaltet werden.
   Schritt 1300:
      Der Positioniertisch wird wieder in die Ladeposition gefahren, um ein sicheres Entnehmen der Mikrotiterplatte(n) zu ermöglichen.
   Schritt 1400:
      Das Programm kann nun beendet werden oder der Nutzer kann zu Schritt 300 zurückkehren, um einen weiteren Lauf zu programmieren.

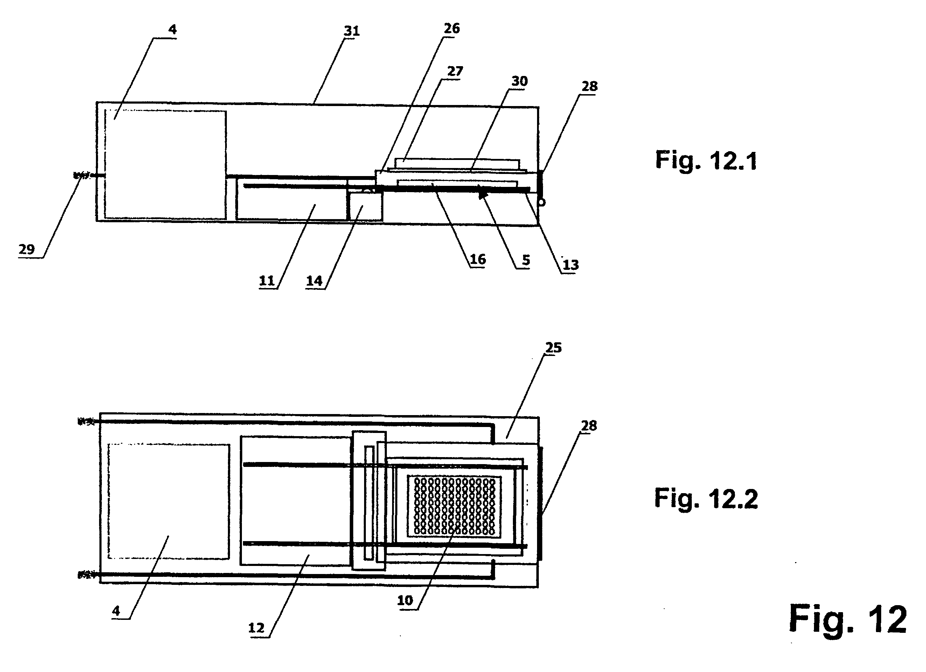

In Fig. 12.1 ist der prinzipielle Gesamtaufbau der CALI-Vorrichtung unter Verwendung einer Beleuchtungskörpereinheit in Seitenansicht dargestellt. Fig. 12.2 stellt die Draufsicht der Gesamtanordnung dar. Analog zu dem Ausführungsbeispiel der CALI-Vorrichtung mit Laservorrichtung gemäß dem ersten Ausführungsbeispiel weist die Vorrichtung gemäß diesem zweiten Ausführungsbeispiel eine Steuereinheit 4, einen Probenhalter 5 mit einem Positioniertisch 13, aufwelchem eine oder auch mehrere Mikrotiterplatten 10 angeordnet sind und welcher zumindest zur Beladung der CALI-Vorrichtung in eine Richtung, vorzugsweise die x-Richtung bewegbar ist, und eine Gleitführung 11 auf, welche mit einem als Antrieb dienenden Schrittmotor 14 verbunden ist. Auf dem Positioniertisch 13 ist ein Träger 16 für die Mikrotiterplatte 10 vorgesehen. Über der Mikrotiterplatte 10 ist eine klimatisierbare Bestrahlungskammer 26 angeordnet, über welche als Lichtquellenvorrichtung eine Beleuchtungskörpereinheit 27 angebracht ist. Die Beleuchtungskörpereinheit 27 kann einfache Glühlampen, Quarzlampen oder herkömmliche Leuchtstoffröhren oder eine Kombination daraus aufweisen. Bei Verwendung von herkömmlichen Glühlampen erfolgt deren Anordnung in einem Array, wobei auch anstelle der Glühlampen Leuchtdioden möglich sind, welche dann entsprechend den zu bestrahlenden Proben eingeschaltet werden können.

Die erfindungsgemäße CALI-Vorrichtung mit Beleuchtungskörpereinheit in Form einer Weißlichtquelle weist eine derartige Anordnung der Lichtquelle auf, daß der gesamte Bereich der Mikrotiterplatte 10 überdeckt ist. Sollen aus Gründen der Untersuchungsabfolge für die einzelnen Proben einzelne auf der Mikrotiterplatte 10 angeordnete Proben bezüglich ihrer Bestrahlung ein- bzw. ausgeblendet werden, so ist es möglich, beispielsweise ferroelektrische LC-Shutter 30 (z.B. Scitec FLC 2436) einzusetzen. Die Steuerung der Shutter 30 erfolgt ebenfalls über ein zur zentralen Steuereinrichtung 4 gehörendes Softwaremodul der zentralen XCALIbur-Software. Die Programmierung dieses Steuermoduls bei Verwendung mehrerer Mikrotiterplatten 10 kann dabei im voraus erfolgen, wobei die einzelnen Programme bis zu deren Abarbeitung speicherbar sind.

Die einzelnen Komponenten der erfindungsgemäßen CALI-Vorrichtung sind in einem ebenfalls klimatisierbaren Gehäuse 25 angeordnet, welcher gegenüber der Umgebung ebenfalls abdichtbar ist und in welchem bestimmte definierte Bedingungen erzeugt werden können. Die Beschickung dieses Gehäuses 25, welches ebenfalls als Inkubator bezeichnet wird, mit einem neuen Set von zu untersuchenden Proben erfolgt über eine in Form einer Klappe 28 in einer Wand des Inkubators 25 angeordneten Öffnung, durch welche der Positioniertisch 13 mittels der durch Servomotoren 14 angetriebenen Gleitführung 11 aus dem Inkubator 25 herausfahrbar ist, mit neuen Mikrotiterplatten beladbar ist und anschließend wieder in den Inkubator 25 einfahrbar ist.

Zur Erhöhung des Durchsatzes ist es selbstverständlich auch möglich, zwei oder mehrere Beleuchtungskörpereinheiten (Bestrahlungsgeräte) 26 parallel anzuordnen und zu betreiben, wobei deren Steuerung ebenfalls von der zentralen Software XCALIbur erfolgt. Diese Steuerungssoftware sorgt für die Koordination der Bewegung der einzelnen Geräte, der Untersuchungen der einzelnen Proben sowie des externen automatischen Transportsystems.

Die erfindungsgemäße CALI-Vorrichtung ist aufgrund des einfachen Aufbaus leicht in eine bestehende Laborautomatisierung einzubinden, wobei die Beschikkung und Entleerung der CALI-Vorrichtung durch zur Gesamtlabor-Automatisisierungsausstattung gehörende entsprechende Transportroboter erfolgen kann (z.B. CRS Linear Robot).

Mit der CALI-Vorrichtung ist das Aufrechterhalten von Zellkulturbedingungen wie beispielsweise Kohlendioxidgehalt, Luftfeuchte und Temperatur während der gesamten Dauer der Bestrahlung möglich. Für bestimmte Untersuchungen ist es des weiteren möglich, flexibel Veränderungen der Zellkulturbedingungen über die Zeit ebenfalls mit einzubeziehen. Des weiteren ist es ebenfalls möglich, definierte Bereiche der Mikrotiterplatte von der Bestrahlung auszunehmen oder mit unterschiedlicher Strahlungsdauer zu bestrahlen. Damit wird die Flexibilität der Untersuchung weiter erhöht, und es werden Kontrollexperimente ohne weiteres möglich.

Aufgrund der Verwendung einer Beleuchtungskörpereinheit 27 ist es somit auf einfache Art und Weise ohne Verwendung eines Lasers möglich, ein effektives, kostengünstiges und zuverlässig arbeitendes Gerät zur Durchführung des CALI-Verfahrens zu realisieren.

### Bezugszeichenliste

- 1: CALI-Vorrichtung
- 2: Laservorrichtung
- 3: Laserstrahl
- 4: Steuereinrichtung
- 4.1.: zentrale Systemsteuersoftware
- 4.2: Lasersteuermodul
- 4.3: Steuermodul für optischen parametrischen Oszillator (XPO)
- 4.4: CNC-Steuermodul
- 5: Probenhalter
- 6: Strahlmodifikationseinrichtung
- 7: Leistungsmesser
- 8: optischer Tisch
- 9: optisches Prisma
- 10: Mikrotiterplatten
- 11: Gleitführung in x-Richtung
- 12: Gleitführung in y-Richtung
- 13: Positioniertisch
- 14: Schrittmotoren
- 15: Kugelspindeln
- 16: Träger / Rezeptor für Mikrotiterplatten
- 17, 18: Stahlplatten
- 19: Schrauben
- 20: Peltier-Elemente
- 21: Durchbruch für Versorgungsleitungen
- 22: Klappe
- 23: Befestigungsöffnungen
- 24: Durchbruch für Bestrahlungsoptik
- 25: Inkubator
- 26: Bestrahlungskammer
- 27: Lichtquellenvorrichtung/Beleuchtungskörpereinheit
- 28: Klappe
- 29: Zuleitungen
- 30: Shutter

## Patentansprüche

1. Vorrichtung (1) für automatisierte chromophor-unterstützte Laser-Inaktivierung (CALI = Chromophore-Assisted Laser Inactivation) mit einer Laservorrichtung (2) zur Bestrahlung von Proben mittels eines Laserstrahls (3) zur Identifikation der Funktion von biologischen Molekülen, mit einer Steuereinrichtung (4), mit zumindest einem Probenhalter (5), wobei mittels eines Antriebs für die Laservorrichtung (2) und/oder den Probenhalter (5) eine Relativbewegung in zumindest zwei Richtungen zwischen dem Laserstrahl (3) und dem Probenhalter (5) derart erzeugbar ist, daß die Proben mit dem Laserstrahl (3) bestrahlbar/abtastbar sind, und mit einem Leistungsmesser (7) zur Bestimmung der von der Laservorrichtung (2) abgegebenen Leistung und zur probenabhängigen Steuerung/Regelung, wobei der Laserstrahl (3) mittels einer Strahlmodifikationseinrichtung (6, 9) in seiner Form und/oder Leistung und/oder Intensität an die Probengröße und/oder die Probenart und/oder die Probenanzahl anpaßbar und die Steuereinrichtung (4) so ausgebildet ist, daß die Laservorrichtung (2) und deren Antrieb und/oder der Antrieb für den Probenhalter (5) und die Strahlmodifikationseinrichtung (6, 9) mittels dieser Steuereinrichtung (4) zentral mittels einer Systemsoftware (4.1) steuerbar sind.

2. Vorrichtung nach Anspruch 1, **dadurch gekennzeichnet, daß** die Strahlmodifikationseinrichtung (6, 9) den Laserstrahl (3) im Querschnitt eindimensional insbesondere als gerade Linie oder zweidimensional als Fläche aufweitet/einengt/begrenzt; insbesondere wobei die Strahlmodifikationseinrichtung (6, 9) den Laserstrahl (3) im Querschnitt rechteckig, kreisförmig oder ellipsenförmig modifiziert.

3. Vorrichtung nach Anspruch 1 oder 2, **dadurch gekennzeichnet, daß** die Strahlmodifikationseinrichtung (6) eine Strahlaufweitvorrichtung (beam expander) ist; oder dadurch, daß die Strahlmodifikationseinrichtung (9) ein Strahlteiler (beam splitter) ist und/oder zusätzlich eine Strahlaufweitvorrichtung vorgesehen ist; insbesondere wobei die Strahlmodifikationseinrichtung (9) so ausgebildet ist, daß zumindest zwei Proben gleichzeitig bestrahlbar sind.

4. Vorrichtung nach einem der Ansprüche 1 bis 3, **dadurch gekennzeichnet, daß** die Laservorrichtung (2) ein gepulster Laser, insbesondere ein Nd:YAG-Infrarotlaser mit einem nachgeschalteten parametrischen Oszillator zur Wellenlängenänderung ist; oder dadurch, daß die Laservorrichtung (2) ein kontinuierlicher Laser, insbesondere ein Argon-Ionen-Gas-Laser, ein Argon-gepumpter justierbarer Farbstofflaser oder ein Helium-Neon-Laser ist.

5. Vorrichtung nach einem der Ansprüche 1 bis 4, **dadurch gekennzeichnet, daß** die Steuereinrichtung (4) den Laserstrahl (3) in seinem Querschnitt, seiner Intensität, Strahlungsdauer und Abmessung in Abhängigkeit von der Bewegung des Probenhalters (5), der Anzahl und/oder der Art der Proben variiert.

6. Vorrichtung (1) für automatisierte Chromophor-unterstützte Licht-Inaktivierung (CALI = Chromophore-Assisted Light Inactivation) mit einer Lichtquellenvorrichtung (27) zur Bestrahlung von Proben mittels Licht zur Identifikation der Funktion von biologischen Molekülen. mit einer Steuereinrichtung (4), mit zumindest einem Probenhalter (5) und mit einem Leistungsmesser (7) zur Bestimmung der von der Lichtquellenvorrichtung (27) abgegebenen Leistung/Lichtausbeute und zur probenabhängigen Steuerung/Regelung, wobei die Lichtquellenvorrichtung (27) eine Beleuchtungskörpereinheit (27) ist, bei welcher zumindest ein Teil der Wellenlänge ihres ausgesendeten Lichts im sichtbaren Bereich liegt, und wobei mittels der Steuereinrichtung die mit dem Leistungsmesser (7) gemessene Leistung/Lichtausbeute einstellbar ist.

7. Vorrichtung (1) nach Anspruch 6, **dadurch gekennzeichnet, daß** das Licht mittels einer Strahlmodifikationseinrichtung (6) in seiner Form und/oder Intensität an die Probengröße und/oder die Probenart und/oder die Probenanzahl anpaßbar ist; insbesondere wobei mittels der Steuereinrichtung (4) ein Antrieb zur Bewegung des Probenhalters (5) und die Strahlmodifikationseinrichtung (6) zentral über eine Systemsoftware (4.1.) steuerbar sind.

8. Vorrichtung nach einem der Ansprüche 6 oder 7, **dadurch gekennzeichnet, daß** die Beleuchtungskörpereinheit (27) zumindest eine Glühlampe, Quarzlampe, Leuchtstoffröhre oder Leuchtdiode, insbesondere in Form einer Weißlichtquelle, aufweist.

9. Vorrichtung nach Anspruch 8, **dadurch gekennzeichnet, daß** die Strahlmodifikationseinrichtung (6) Masken, insbesondere mit variablen Öffnungen/Blenden, aufweist.

10. Vorrichtung nach einem der Ansprüche 6 bis 9, **dadurch gekennzeichnet, daß** das Licht diffuses Licht ist; insbesondere dadurch, daß das Licht zumindest ein Lichtstrahl ist.

11. Vorrichtung nach einem der Ansprüche 1 bis 10, **dadurch gekennzeichnet, daß** die Lichtquellenvorrichtung (27) so ausgebildet ist, daß auf dem Probenhalter (5) angeordnete Proben gleichzeitig bestrahlbar sind.

12. Vorrichtung nach einem der Ansprüche 1 bis 11, **dadurch gekennzeichnet, daß** der Probenhalter (5) einen Positioniertisch (13) aufweist, welcher zumindest eine Mikrotiterplatte (10), insbesondere mit Kavitäten unterschiedlicher Abmessungen, trägt und mittels rechtwinklig zueinander angeordneter, als Antrieb Schrittmotoren (14) aufweisender Verschiebemechanismen (11, 12) in x, y-Richtung positionierbar ist.

13. Vorrichtung nach Anspruch 12, **dadurch gekennzeichnet, daß** die Verschiebemechanismen (11, 12) schienengeführt sind und Kugelspindeln (15) zur Übertragung der Bewegung vom jeweiligen Schrittmotor (14) aufweisen.

14. Vorrichtung nach einem der Ansprüche 1 bis 13, **dadurch gekennzeichnet, daß** der Probenhalter (5) zwei Mikrotiterplatten (10) trägt.

15. Vorrichtung nach einem der Ansprüche 1 bis 14, **dadurch gekennzeichnet, daß** eine Ligandenbindungspartner(LBP)-Screening-Maschine zur Erzeugung spezifischer, gegen spezielle Zielmolekül-Liganden gerichtete LBPs, eine Vorrichtung zur Produktion/Reinigung von BPs, eine LBP-Chromophor-Kopplungsvorrichtung zur Verknüpfung der ausgewählten LBPs und der synthetisierten Chromophore, eine Beladungsvorrichtung zum Überführen von LBP-Tags in in der Mikrotiterplatte (10) angeordnete Kavitäten, eine Einrichtung zum Bewegen der Mikrotiterplatte (10) in die CALI-Vorrichtung (1) und eine Vorrichtung zum Ablesen von Aktivitätsergebnissen vorgesehen sind.

16. Vorrichtung nach Anspruch 15, **dadurch gekennzeichnet, daß** die abgelesenen Aktivitätsergebnisse in einer Datenbank abspeicherbar sind.

17. Vorrichtung nach Anspruch 15 oder 16, **dadurch gekennzeichnet, daß** die Screening-Maschine, die LBP-Chromophore-Kopplungsvorrichtung, die Beladungsvorrichtung und die Einrichtung zum Bewegen der Mikrotiterplatte (10) mittels der Steuereinrichtung (4) über die Systemsoftware (4.1) zentral steuerbar sind.

18. Vorrichtung nach einem der Ansprüche 12 bis 17, **dadurch gekennzeichnet, daß** ein Inkubator (25) zumindest eine Mikrotiterplatte (10) des Probenhalters (5) umschließt, welches mit äußeren Versorgungssystemen derart verbunden ist, daß vorher eingestellte Zustandsbedingungen, insbesondere Kohlendioxid, Feuchtigkeit, Temperatur, Druck, steuerbar sind; insbesondere wobei die Versorgungssysteme mittels der Steuereinrichtung (4) über die Systemsoftware (4.1) zentral steuerbar sind.

19. Verfahren zur Identifikation der Funktion eines Liganden L, bei welchem eine Probe mit einem Komplex aus einem Liganden und einem Ligandenbindungspartner(LBP)-lichtaktivierbaren-Marker (Tag) mit Licht, ausgesendet von einem Beleuchtungskörper, bestrahlt wird, dessen Leistung gemessen und probenabhängig gesteuert/geregelt wird, wobei mindestens ein Teil der Wellenlänge seines ausgesendeten Lichts im sichtbaren Bereich liegt.

20. Verfahren nach Anspruch 19, wobei der Beleuchtungskörper eine Weißlichtquelle wie eine Glühlampe, Quarzlampe, Leuchtstoffröhre oder Leuchtdiode ist.

21. Verfahren nach Anspruch 19 oder 20, **gekennzeichnet durch** die Stufen:
a) Auswählen eines Ligandenbindungspartners (LBP) mit Spezifität für den Liganden L,
b) Koppeln des LBP an einen lichtaktivierbaren Marker (Tag) unter Bildung von LBP-Tag, gegebenenfalls nach vorheriger Modifikation des LBP im Hinblick auf eine effizientere Bindung an den Marker,
c) Inkontaktbringen des Liganden L mit mindestens einem LBP-Tag unter Bildung eines L/LBP-Tag-Komplexes, und
d) Bestrahlen des L/LBP-Tag-Komplexes unter Messung/Einstellung der Leistung einer Lichtquellenvorrichtung, wobei der bestrahlte LBP-Tag selektiv den gebundenen Liganden modifiziert,
wobei die Reihenfolge der Stufen b) und c) vertauscht werden kann.

22. Verfahren nach einem der Ansprüche 19-21, wobei das ausgesendete Licht mindestens ein Lichtstrahl ist.

23. Verfahren nach einem der Ansprüche 19-21, wobei die Weißlichtquelle diffuses Licht zur Bestrahlung einer Vielzahl von Proben aussendet.

24. Verfahren nach einem der Ansprüche 19-23, wobei die Vielzahl von Proben gleichzeitig bestrahlt wird und in einer oder mehreren Mikrotiterplatten angeordnet sind, die auf dem Probenhalter (5) angeordnet ist/sind.

25. Verfahren nach einem der Ansprüche 19-24, wobei die Vielzahl von Proben mit einer oder mehreren Beleuchtungskörpereinheiten (26) bestrahlt wird, die parallel angeordnet und betrieben werden, wobei deren Steuerung über die zentrale Software (4.1) erfolgt.

26. Verfahren nach einem der Ansprüche 19-25, wobei der LBP ausgewählt ist aus dsFv (disulfid-linked variable chain fragment), scFv (single chain variable fragment), Fab (fragment, antigen-binding), Diabody, immunglobulinartigen Molekülen, Peptiden, RNA, DNA, PNA und kleinen organischen Molekülen; ausgenommen intakte Antikörpermoleküle.

27. Verfahren nach einem der Ansprüche 19-26, wobei der LBP aus einer kombinatorischen Bank stammt; ausgenommen Hybridome.

## Claims

1. Apparatus (1) for automated chromophore-assisted laser inactivation (CALI) having a laser device (2) for the irradiation of samples by means of a laser beam (3) for identifying the function of biological molecules, having a control unit (4) and having at least one sample holder (5), where a relative movement in at least two directions between the laser beam (3) and the sample holder (5) can be produced by means of a drive for the laser device (2) and/or the sample holder (5) in such a way that the samples can be irradiated/scanned by the laser beam (3), and having a power meter (7) for determining the power emitted by the laser device (2) and for sample-dependant control, whereby said laser beam (3) can be matched in shape and/or power and/or intensity to the sample size and/or the sample type and/or the sample number by means of a beam modification device (6, 9), and the control unit (4) is designed in such a way that the laser device (2) and its drive and/or the drive for the sample holder (5) and the beam modification device (6, 9) can be controlled centrally by this control unit (4) by means of system software (4.1).

2. Apparatus according to claim 1, **characterized in that** the beam modification device (6,9) expands/narrows/limits the laser beam (3) one-dimensionally in cross section, in particular as a straight line, or two-dimensionally as an area; in particular whereby the beam modification device (6,9) modifies the laser beam (3) in cross section in a rectangular, circular or elliptical manner.

3. Apparatus according to claims 1 or 2, **characterized in that** the beam modification device (6) is a beam expander; or that the beam modification device (9) is a beam splitter and/or a beam expander is additionally provided; in particular whereby the beam modification device (9) is designed in such a way that at least two samples can be irradiated simultaneously..

4. Apparatus according to any of claims 1-3, in which the laser device (2) is a pulsed laser, in particular an Nd:YAG infrared laser having a downstream parametric oscillator for wavelength modification; or wherein the laser device (2) is a continuous laser; in particular an argon-ion laser, an adjustable argon-pumped dye laser or a helium-neon laser.

5. Apparatus according to any of claims 1-4, in which the control unit (4) varies the laser beam (3) in cross section, intensity, irradiation duration and dimensions as a function of the movement of the sample holder (5), the number and/or the type of the samples.

6. Apparatus (1) for automated chromophore-assisted light inactivation (CALI= Chromophore-Assisted Light Inactivation), having a light-source device (27) for irradiating samples by means of light for identifying the function of biological molecules, having a control unit (4), and having at least one sample holder (5), and a power meter (7) for determining the power/light yield emitted by the light-source device (27) and for sample-dependant control, whereby said light-source device (27) is a lamp unit (27) in which at least part of the wavelength of its emitted light is in the visible region, and wherein the power/light yield measured with the power meter (7) is adjustable by means of the control unit.

7. Apparatus (1) according to claim 6, **characterized in that** the light can be matched in shape and/or intensity to the sample size and/or the sample type and/or the sample number by means of a beam modification device (6); in particular whereby a drive for moving the sample holder (5) and the beam modification device (6) can be controlled centrally via system software (4.1) by means of the control unit (4).

8. Apparatus according to claims 6 or 7, **characterized in that** the lamp unit (27) has at least one incandescent lamp, quartz lamp, fluorescent tube or light-emitting diode, in particular in the form of a white-light source.

9. Apparatus according to claim 8, **characterized in that** the beam modification device (6) has masks, in particular having variable apertures/diaphragms.

10. Apparatus according to any of claims 6-9, in which the light is diffuse light, in particular whereby the light is at least one light beam.

11. Apparatus according to any of claims 1-10, in which the light-source device (27) is designed in such a way that samples arranged on the sample holder (5) can be irradiated simultaneously.

12. Apparatus according to any of claims 1-11, in which the sample holder (5) has a positioning stage (13) which carries at least one microtiter plate (10), in particular having wells of different dimensions, and can be positioned in the x, y direction by means of displacement mechanisms (11, 12) which are arranged at right angles to one another and which have stepping motors (14) as drive.

13. Apparatus according to claim 12, **characterized in that** the displacement mechanisms (11, 12) are guided on rails and have ball spindles (15) for transmission of the movement from the respective stepping motor (14).

14. Apparatus according to any of claims 1-13, in which the sample holder (5) carries two microtiter plates (10).

15. Apparatus according to any of claims 1-14, in which a ligand-binding partner (LBP) screening machine for generating specific LBPs directed against specific target molecule ligands, a unit for the production/purification of BPs, and an LBP-chromophore coupling unit for linking the selected LBPs and the synthesized chromophores, a loading device for transferring LBP tags into wells arranged in the microtiter plate (10), a device for moving the microtiter plate (10) into the CALI apparatus (1), and a device for reading activity results are provided.

16. Apparatus according to claim 15, **characterized in that** the activity results read can be stored in a data base.

17. Apparatus according to claims 15 or 16, **characterized in that** the screening machine, the LBP-chromophore coupling unit, the loading device and the device for moving the microtiter plate (10) can be controlled centrally by means of the control unit (4) via the system software (4.1).

18. Apparatus according to any of claims 12-17, in which an incubator (25) surrounds at least one microtiter plate (10) of the sample holder (5) and is connected to external supply systems in such a way that preset state conditions, in particular carbon dioxide, moisture, temperature and pressure, can be controlled; in particular whereby the supply systems can be centrally controlled by means of the control unit (4) via the system software (4.1).

19. Process for identifying the function of a ligand L, in which a sample containing a complex of a ligand and a ligand-binding partner (LBP)-light-activatable tag is irradiated with a light beam from a lamp unit, whereby the power is measured and adjusted in a sample dependant manner, whereby at least part of the wavelength of its emitted light is in the visible region.

20. Process according to claim 19, **characterized in that** the lamp unit is a white light source selected from the group consisting of an incandescent lamp, a quartz lamp, a fluorescent tube and a light-emitting diode.

21. Process according to claims 19 or 20, **characterized by** the steps:
a) selecting a ligand-binding partner (LBP) having specificity for the ligand L,
b) coupling of the LBP to a light-activatable tag with formation of LBP-tag, if necessary after prior modification of the LBP in respect of efficient binding to the tag,
c) bringing the ligand L into contact with at least one LBP-tag with formation of an L/LBP-tag complex, and
d) irradiation of the L/LBP-tag complex while measuring/adjusting the power of a light source device, where the irradiated LBP-tag modifies the bound ligands selectively,
where the sequence of steps b) and c) can be interchanged.

22. Process according to any of claims 19-21, in which the emitted light is at least one light beam.

23. Process according to any of claims 19-21, in which the white light source emits diffuse light for irradiating a plurality of samples.

24. Process according to any of claims 19-23, in which the plurality of samples is irradiated simultaneously, and is arranged in one or more microtiter plates which are arranged on the sample holder (5).

25. Process according to any of claims 19-24, in which the plurality of samples is irradiated with one or more light-source devices (27), whereby said light source devices are arranged and controlled in parallel and are centrally controlled via the system software (4.1).

26. Process according to any of claims 19-25, in which the LBP is selected from from the group consisting of dsFv (disulfid-linked variable chain fragment), scFv (single chain variable fragment), Fab (fragment, antigen-binding), diabody, immunoglobulin-like molecules, peptides, RNA, DNA, PNA, and small organic molecules, except intact antibody molecules.

27. Process according to any of claims 19-26, in which the LBP is derived from a combinatorial library, excepting hybridomas.

## Revendications

1. Appareillage (1) pour l'inactivation automatisée par laser assistée par un chromophore (CALI = Chromophore-Assisted Laser Inactivation), comportant un dispositif à laser (2) pour irradier des échantillons à l'aide d'un faisceau laser (3) pour identifier la fonction de molécules biologique, un dispositif de commande (4) comportant au moins un porte-échantillon (5), auquel cas, à l'aide d'un entraînement pour le dispositif laser (2) et/ou le porte-échantillon (5), il est possible de produire un mouvement relatif dans au moins deux directions entre le faisceau laser (3) et le porte-échantillon (5), de façon que les échantillons puissent être irradiés/explorés par le faisceau laser (3), ainsi qu'un wattmètre (7), destiné à mesurer la puissance cédée par le dispositif laser (2) et pour assurer une commande/une régulation qui dépendent de l'échantillon, le faisceau laser (3) pouvant, par un dispositif (6, 9) de modification du faisceau, être adapté, pour ce qui est de sa forme et/ou de sa puissance et/ou de son intensité, à la taille et/ou à la nature et/ou au nombre des échantillons, et le dispositif de commande (4) étant configuré de telle sorte que le dispositif laser (2) et son entraînement, et/ou l'entraînement du porte-échantillon (5) et le dispositif (6, 9) de modification du faisceau, puissent être commandés dune manière centralisée à l'aide de ce dispositif de commande (4), au moyen d'un logiciel système (4.1).

2. Appareillage selon la revendication 1, **caractérisé en ce que** le dispositif (6, 9) de modification du faisceau élargit/rétrécit/limite la section transversale du faisceau laser (3), d'une manière monodimensionnelle en particulier sous forme d'une ligne droite, ou d'une manière bidimensionnelle sous forme d'une surface ; en particulier **en ce que** le dispositif (6, 9) de modification du faisceau modifie le faisceau laser (3) en lui donnant une section transversale rectangulaire, circulaire ou elliptique.

3. Appareillage selon la revendication 1 ou 2, **caractérisé en ce que** le dispositif (6) de modification du faisceau est un dispositif élargisseur de faisceau (beam expander) ; ou **en ce que** le dispositif (9) de modification du faisceau est un diviseur de faisceau (beam splitter) et/ou que l'on prévoit en outre un dispositif élargisseur de faisceau ; en particulier **en ce que** le dispositif (9) de modification du faisceau est configuré de façon à pouvoir irradier simultanément au moins deux échantillons.

4. Appareillage selon l'une des revendications 1 à 3, **caractérisé en ce que** le dispositif laser (2) est un laser pulsé, en particulier un laser infrarouge Nd:YAG, un oscillateur paramétrique, destiné à modifier la longueur d'onde, étant installé en aval ; ou **en ce que** le dispositif laser (2) est un laser continu, en particulier un laser argon-ions-gaz, un laser ajustable à colorant, avec pompage d'argon, ou un laser à hélium-néon.

5. Appareillage selon l'une des revendications 1 à 4, **caractérisé en ce que** le dispositif de commande (4) fait varier la section transversale, l'intensité, la durée du rayonnement et les dimensions du faisceau laser (3) en fonction du déplacement du porte-échantillon (5), du nombre et/ou de la nature des échantillons.

6. Appareillage (1) pour l'inactivation aitomatisée par la lumière assistée par un chromophore (CALI = Chromophore-Assisted Light Inactivation), avec un dispositif (27) formant source lumineuse, destiné à irradier des échantillons à l'aide d'une lumière, pour identifier la fonction de molécules biologiques, avec un dispositif de commande (4), avec au moins un porte-échantillon et au moins un wattmètre (7) destiné à déterminer la puissance/le rendement lumineux émis par le dispositif (27) formant source lumineuse, et pour assurer une commande/régulation dépendant de l'échantillon, le dispositif (27) formant source lumineuse étant une unité d'illuminant (27), où au moins une partie de la longueur d'onde de la lumière émise par cette dernière se trouve dans le domaine visible, la puissance/le rendement lumineux mesuré à l'aide du wattmètre (7) pouvant être ajusté à l'aide du dispositif de commande.

7. Appareillage (1) selon la revendication 6, **caractérisé en ce que** la lumière peut, à l'aide d'un dispositif (6) de modification du faisceau, être adaptée, pour ce qui est de sa forme et/ou de son intensité, à la taille et/ou à la nature et/ou au nombre des échantillons ; et en particulier **en ce que**, à l'aide du dispositif de commande (4), un entraînement destiné à déplacer le porte-échantillon (5) et le dispositif (6) de modification du faisceau peut être commandé d'une manière centralisée par l'intermédiaire d'un logiciel système (4.1).

8. Appareillage selon l'une des revendications 6 ou 7, **caractérisé en ce que** l'unité d'illuminant (27) comporte au moins une lampe à incandescence, une lampe à quartz, un tube luminescent ou une diode électroluminescente, en particulier sous forme d'une source de lumière blanche.

9. Appareillage selon la revendication 8, **caractérisé en ce que** le dispositif (6) de modification du faisceau comporte des masques, en particulier avec des ouvertures/diaphragmes variables.

10. Appareillage selon l'une des revendications 6 à 9, **caractérisé en ce que** la lumière est une lumière diffuse ; et en particulier **en ce que** la lumière est au moins un faisceau lumineux.

11. Appareillage selon l'une des revendications 1 à 10, **caractérisé en ce que** le dispositif (27) formant source lumineuse est configuré de façon que des échantillons disposés sur le porte-échantillon (5) puissent être irradiés simultanément.

12. Appareillage selon l'une des revendications 1 à 11, **caractérisé en ce que** le porte-échantillon (5) comporte une table de positionnement (13), qui porte au moins une plaque de microtitrage (10), ayant en particulier des puits ayant des dimensions différentes, et qui peut être positionnée dans la direction x, y à l'aide de mécanismes de déplacement (11, 12), disposés perpendiculairement l'un à l'autre et comportant des moteurs pas à pas (14) servant d'entraînement.

13. Appareillage selon la revendication 12, **caractérisé en ce que** les mécanismes de déplacement (11, 12) sont guidés par des rails et comportent des vis à billes (15), pour le transfert du déplacement à partir du moteur pas à pas (14) correspondant.

14. Appareillage selon l'une des revendications 1 à 13, **caractérisé en ce que** le porte-échantillon (5) porte deux plaques de microtitrage (10).

15. Appareillage selon l'une des revendications 1 à 14, **caractérisé en ce que** l'on prévoit une machine pour le criblage de partenaires de liaison des ligands (LBP), destinée à produire des LBP spécifiques, dirigés contre des molécules cibles spéciales de ligands, un équipement pour la production/la purification des BP, un dispositif de couplage LBP-chromophore, destiné à relier les LBP sélectionnés et les chromophores synthétisés, un dispositif de chargement, pour transférer des marqueurs de LBP dans les puits disposés dans la plaque de microtitrage (10), un dispositif pour déplacer la plaque de microtitrage (10) dans l'équipement CALI (1), et un équipement pour lire les résultats des essais d'activité.

16. Appareillage selon la revendication 15, **caractérisé en ce que** les résultats lus des essais d'activité peuvent être mémorisés dans une banque de données.

17. Appareillage selon la revendication 15 ou 16, **caractérisé en ce que** la machine de criblage, le dispositif de couplage LBP-chromophore, le dispositif de chargement et le dispositif permettant le déplacement de la plaque de microtitrage (10), peuvent être commandés d'une manière centralisée à l'aide du dispositif de commande (4) par l'intermédiaire du logiciel système (4.1).

18. Appareillage selon l'une des revendications 12 à 17, **caractérisé en ce qu'**un incubateur (25) comporte au moins une plaque de microtitrage (10) du porte-échantillon (5), qui est relié à des systèmes d'alimentation extérieurs de façon à permettre la commande de conditions d'état ajustées au préalable, notamment le dioxyde de carbone, l'humidité, la température, la pression, et en particulier **en ce que** les systèmes d'alimentation peuvent être commandés d'une manière centralisée à l'aide du dispositif de commande (4) par l'intermédiaire du logiciel système (4.1).

19. Procédé pour identifier la fonction d'un ligand L, dans lequel un échantillon comportant un complexe d'un ligand et d'un marqueur (Tag) activable à la lumière d'un partenaire de liaison des ligands (LBP) est irradié par une lumière émise par un illuminant, dont la puissance est mesurée, et commandée/régulée en fonction de l'échantillon, au moins une partie de la longueur d'onde de sa lumière émise se trouvant dans le domaine visible.

20. Procédé selon la revendication 19, dans lequel l'illuminant est une source de lumière blanche telle qu'une lampe à incandescence, une lampe à quartz, un tube luminescent ou une diode électroluminescente.

21. Procédé selon la revendication 19 ou 20, **caractérisé par** les étapes suivantes :
a) sélection d'un partenaire de liaison des ligands (LBP) ayant une spécificité vis-à-vis du ligand L,
b) couplage du LBP à un marqueur (Tag) photoactivable, avec formation d'un marqueur de LBP, éventuellement après une modification préalable du LBP, eu égard à une liaison plus efficace au marqueur,
c) mise en contact du ligand L avec au moins un marqueur de LBP, avec formation d'un complexe L/marqueur de LBP, et
d) irradiation du complexe L/marqueur de LBP, avec mesure/ajustement de la puissance d'un équipement formant source lumineuse, le marqueur de LBP irradié modifiant d'une manière sélective le ligand lié,
la séquence des étapes b) et c) pouvant être inversée.

22. Procédé selon l'une des revendications 19 à 21, dans lequel la lumière émise est au moins un faisceau lumineux.

23. Procédé selon l'une des revendications 19 à 21, dans lequel la source de lumière blanche émet une lumière diffuse pour irradier un grand nombre d'échantillons.

24. Procédé selon l'une des revendications 19 à 23, dans lequel le grand nombre d'échantillons est irradié simultanément et est disposé dans une ou plusieurs plaques de microtitrage, qui sont disposées sur le porte-échantillon (5).

25. Procédé selon l'une des revendications 19 à 24, dans lequel le grand nombre d'échantillons est irradié par une ou plusieurs unités d'illuminant (27), qui sont disposées et exploitées en parallèle, leur commande ayant lieu d'une manière centralisée par l'intermédiaire du logiciel (4.1).

26. Procédé selon l'une des revendications 19 à 25, dans lequel le LBP est choisi parmi le dsFv (fragment de chaîne variable à liaison disulfure), le scFv (fragment variable de chaîne unique), le Fab (fragment, liaison à l'antigène), un Diabody, les molécules de type immunoglobuline, les peptides, l'ARN, l'ADN, l'APN, et les petites molécules organiques ; à l'exception des molécules d'anticorps intactes.

27. Procédé selon l'une des revendications 19 à 26, dans lequel le LBP provient d'une banque combinatoire, à l'exclusion des hybridomes.
